# EUROPEAN PATENT SPECIFICATION

(11) **EP 3 436 198 B1**
(45) Date of publication and mention of the grant of the patent: **04.05.2022**
(21) Application number: 17776453.7
(22) Date of filing: 28.03.2017
(51) Int. Cl.: C12Q 1/6844, G01N 33/487, B01L 3/00

(54) **SURFACE-BASED DETECTION OF NUCLEIC ACID IN A CONVECTION FLOW FLUIDIC DEVICE**
OBERFLÄCHENBASIERTE DETEKTION VON NUKLEINSÄURE IN EINER FLUIDISCHEN KONVEKTIONSSTRÖMUNGSVORRICHTUNG
DÉTECTION DE SURFACE D'ACIDE NUCLÉIQUE DANS UN DISPOSITIF FLUIDIQUE À ÉCOULEMENT PAR CONVECTION

(30) Priority: 29.03.2016 US 201662314909 P
(43) Date of publication of application: 06.02.2019
(60) Divisional application: 22156862.9
(73) Proprietor: William Marsh Rice University, Houston, TX 77005 (US)
(72) Inventor: KHODAKOV, Dmitriy, A, Houston, TX 77005 (US); ZHANG, David, Houston, TX 77030 (US)
(74) Representative: Potter Clarkson
(86) International application number: PCT/US2017/024530
(87) International publication number: WO 2017/172760

(56) References cited:
- WO-A1-2015/037255
- US-A1- 2010 056 397
- US-A1- 2013 274 135
- US-B2- 8 114 636
- YUEHUA GUO ET AL: "Target-driven DNA association to initiate cyclic assembly of hairpins for biosensing and logic gate operation", CHEMICAL SCIENCE, vol. 6, no. 7, 1 January 2015 (2015-01-01) , pages 4318-4323, XP55625113, United Kingdom ISSN: 2041-6520, DOI: 10.1039/C5SC01215E

## Description

### FIELD

The disclosure describes novel reagents, instruments, and methods for detection and quantitation of specific nucleic acid sequences for scientific and clinical research and diagnostics applications.

### BACKGROUND

Most commercial nucleic acid (NA) assays require the use of enzymes for molecular or signal amplification. Enzymes such as DNA polymerase have been optimized to be fast and specific. Reconstitution of lyophilized enzymes in resource-limited conditions reduces the need for a cold chain. Isothermal nucleic acid amplification assays such as NEAR, LAMP and NASBA enable DNA/RNA profiling without complex temperature cycling equipment. Despite these many advances, existing nucleic acid detection technologies still face challenges for rapid PoC (point of care) detection of pathogen biomarkers, because it is difficult to design/evolve enzymes that simultaneously capture all desirable properties (e.g., fast, high fidelity, and robust to chemicals/inhibitors).

A number of existing nucleic acid analysis technologies are enzyme-free, including microarrays, fluorescence *in situ* hybridization (FISH), branched DNA dendrimers (Panomics), and fluorescent barcoding (Nanostring). In these approaches, the DNA or RNA target molecules stoichiometrically recruit or are converted into a limited number of fluorescent groups. This is unlike PCR where even a single nucleic acid molecule is amplified endlessly to produce an arbitrarily high number of amplicon molecules. Consequently, expensive and bulky equipment is needed for these approaches to achieve the molecular sensitivity needed to detect and analyze the small amounts of DNA or RNA target present in biological samples, restricting their use for PoC applications and in the limited resources conditions.

Another group of nucleic acid identification techniques employs solution-based enzyme-free DNA amplification approaches. Here a single-stranded DNA target molecule can catalytically release DNA oligonucleotides with identical sequence to the target from the preassembled DNA detection complexes in unlimited manner. Clinically relevant limits of detection have yet to be demonstrated for this family of approaches. In these systems, there is false positive amplification due to DNA "breathing" events that result in release of amplicon molecules in the absence of the detection of a target sequence.

### SUMMARY

Thus, in accordance with the present disclosure, there is provided a device which is not claimed, comprising a surface having a plurality oligonucleotide complexes, wherein said oligonucleotide complexes each comprise:
a first oligonucleotide comprising a first DNA sequence and a linking moiety for irreversibly linking the first oligonucleotide to the surface, and
a second oligonucleotide comprising a second DNA sequence and a third DNA sequence, wherein the second DNA sequence is complementary to the first DNA sequence and is hybridized thereto, wherein said second DNA oligonucleotide does not comprise a fluorescent moiety and not irreversibly linked to the surface.

Each of said second DNA sequences may be identical, or may not be identical. The plurality of said oligonucleotide complexes may be located in spatially discrete regions on said surface. The first oligonucleotides may comprise a fluorescent moiety, and each of said second oligonucleotides may comprise a fluorescence quencher. Each of said spatially discrete regions may further comprise a third oligonucleotide comprising a fourth DNA sequence and a fifth DNA sequence, wherein the fourth DNA sequence is complementary to the third DNA sequence. The third oligonucleotides may each comprise a fluorescence quencher moiety. The linking moiety may comprise an alkyne group including a strained alkyne or an azide group.

Each of the first DNA sequences may have a length of between about 15 and about 80 nucleotides. Each of the third DNA sequences may have a length of between about 5 and about 80 nucleotides, or a length of between about 15 and about 80 nucleotides. Each of the fifth DNA sequences may have a length of between about 5 and about 20 nucleotides. Each of the fourth DNA sequences may have a length of between about 5 and about 80 nucleotides.

In another embodiment, there is provided a fluidic reaction chamber comprising:
a first surface,
a second surface that does not contact the first surface, wherein said first and second surfaces face each other,
a material contacting the first surface and the second surface and that forms an outer boundary of said reaction chamber, and
a material contacting the first surface and the second surface and that forms and inner boundary of said reaction chamber,
wherein the first surface comprises a plurality of oligonucleotide complexes, wherein said oligonucleotide complexes each comprise:
   a first oligonucleotide comprising a first DNA sequence and a linking moiety for irreversibly linking the first oligonucleotide to the surface, and
   a second oligonucleotide comprising a second DNA sequence and a third DNA sequence, wherein the second DNA sequence is complementary to the first DNA sequence and is hybridized thereto, wherein said second DNA oligonucleotide is not irreversibly linked to the first surface, and optionally does not comprise a fluorescent moiety.

Each of said second DNA sequences may be identical or may be not identical. Each of said plurality of said oligonucleotide complexes may be located in spatially discrete regions on said surface. Each of said first oligonucleotides may comprise a fluorescent moiety. Each of said second oligonucleotides may comprise a fluorescence quencher moiety. Each of said spatially discrete regions further may comprise a third oligonucleotide comprising a fourth DNA sequence and a fifth DNA sequence, wherein the fourth DNA sequence is complementary to the third DNA sequence. The third oligonucleotides may each comprise a fluorescence quencher moiety. The linking moiety may comprise an alkyne group including a strained alkyne or an azide group. The fluidic reaction chamber may have a first port to deliver the sample, and optionally a second another port to allow air/fluid exit when sample is introduced into the first port.

Each of the first DNA sequences may have a length of between about 15 and about 80 nucleotides. Each of the third DNA sequences may have a length of between about 5 and about 80 nucleotides. Each of the third DNA sequences may have a length of between about 15 and about 80 nucleotides. Each of the fifth DNA sequences may have a length of between about 5 and about 20 nucleotides. Each of the fourth DNA sequences may have a length of between about 5 and about 80 nucleotides. The materials contacting first and second surfaces that form the inner and outer boundaries of the chamber may have thickness between 40 microns (40 µm) and 2 millimeters (2 mm).

The fluidic reaction chamber may be circular, oval, square, rectangular, triangular, hexagonal, octagonal, rhomboid or trapezoid, or annular as defined herein. The fluidic reaction chamber may not be at a uniform temperature, and the warmest region of the reaction chamber may be at least 10 °C higher than the coldest region of the reaction chamber. The coldest region of the reaction chamber may be between about 50 °C and about 75 °C. The hottest region of the reaction chamber may be between about 80 °C and about 100 °C. The fluidic reaction chamber may further comprise a fluid disposed within the fluidic reaction chamber, said fluid solution comprising a DNA polymerase, dNTPs, and PCR buffer.

In yet another embodiment, there is provided a method of amplifying a target nucleic acid comprising (a) providing a fluidic reaction chamber according to claim 16, wherein said fluidic reaction chamber is in operable relationship to a first and a second heat source, wherein said first and second heat sources are capable of applying differing first and a second heat levels to said annular chamber, wherein said first and second heat levels are not the same; (b) introducing into said fluidic reaction chamber a fluid comprising a target nucleic acid sequence, a DNA polymerase, dNTPs and a polymerase chain reaction (PCR) buffer; and (c) applying first and second heat levels to said fluidic reaction chamber. The method may further comprise detecting amplification of said target nucleic acid.

Each of said second DNA sequences may be identical or may not be identical. The plurality of said oligonucleotide complexes may be located in spatially discrete regions on said surface. Each of said first oligonucleotides may comprise a fluorescent moiety, and each of said second oligonucleotides may comprise a fluorescence quencher moiety. Each of said spatially discrete regions may further comprise a third oligonucleotide comprising a fourth DNA sequence and a fifth DNA sequence, wherein the fourth DNA sequence is complementary to the third DNA sequence. The third oligonucleotides may each comprise a fluorescence quencher moiety. The linking moiety may comprise an alkyne group including a strained alkyne or an azide group.

The first DNA sequences may have a length of between about 15 and about 80 nucleotides. Each of the third DNA sequences may have a length of between about 5 and about 80 nucleotides. Each of the third DNA sequences may have a length of between about 15 and about 80 nucleotides. Each of the fifth DNA sequences have a length of between about 5 and about 20 nucleotides. The fluidic reaction chamber may be circular, oval, square, triangular, rectangular, hexagonal, octagonal, rhomboid or trapezoid.

The fluidic reaction chamber may not at a uniform temperature, and the warmest region of the reaction chamber may be at least 10 °C higher than the coldest region of the reaction chamber. The coldest region of the reaction chamber may be between about 50 °C and about 75 °C. The hottest region of the reaction chamber may be between about 80 °C and about 100 °C.

In yet a further embodiment, there is provided a device which is not claimed, comprising a first surface region and a second surface region,
the first surface region comprising
   a first oligonucleotide comprising a first DNA sequence and a linking moiety for irreversibly linking the first oligonucleotide to the first surface region, and
   a second oligonucleotide comprising a second DNA sequence and a third DNA sequence, wherein the second sequence is complementary to the first sequence, and
the second surface region comprising
   a third oligonucleotide comprising a fourth DNA sequence and a linking moiety for irreversibly linking the third oligonucleotide to the second surface region, and
   wherein the fourth sequence is complementary to the second sequence, the third sequence, or a combination of at least six continuous nucleotides of the second sequence and six continuous nucleotides of the third sequence, or a device comprising a first surface region and a second surface region,
the first surface region comprising
   a first oligonucleotide comprising a first DNA sequence and a linking moiety for irreversibly linking the first oligonucleotide to the first surface region, and
   a second oligonucleotide comprising a second DNA sequence and a third DNA sequence, wherein the second sequence is complementary to the first sequence, and
the second surface region comprising
   a third oligonucleotide comprising a fourth DNA sequence and a linking moiety for irreversibly linking the third oligonucleotide to the second surface region, and
   a fourth oligonucleotide comprising a fifth DNA sequence and a sixth DNA sequence, wherein the fifth sequence is complementary to the fourth sequence, and
      wherein the second sequence is complementary to the fifth sequence or is complementary to the sixth sequence.

The first or second oligonucleotide may comprises a fluorescent moiety, and the first or second oligonucleotide may comprise a fluorescence quencher. The second DNA sequences may be identical or may not be identical. The plurality of said oligonucleotide complexes may be located in spatially discrete regions on said surface. The linking moiety may comprise an alkyne group including a strained alkyne or an azide group. The fluidic reaction chamber may have a first port to deliver the sample, and optionally a second another port to allow air/fluid exit when sample is introduced into the first port.

Each of the first DNA sequences may have a length of between about 15 and about 80 nucleotides. Each of the third DNA sequences may have a length of between about 5 and about 80 nucleotides. Each of the third DNA sequences may have a length of between about 15 and about 80 nucleotides. Each of the fifth DNA sequences may have a length of between about 5 and about 20 nucleotides. Each of the fourth DNA sequences may have a length of between about 5 and about 80 nucleotides. Each of the sixth DNA sequences may have a length of between 5 and 80 nucleotides.

In still a further embodiment, there is provided a fluidic reaction chamber comprising:
a first surface,
a second surface that does not contact the first surface, wherein said first and second surfaces face each other,
a material contacting the first surface and the second surface and that forms an outer boundary of said reaction chamber, and
a material contacting the first surface and the second surface and that forms and inner boundary of said reaction chamber, wherein
   (a) the first surface comprises
      a first oligonucleotide comprising a first DNA sequence and a linking moiety for irreversibly linking the first oligonucleotide to the first surface region, and
      a second oligonucleotide comprising a second DNA sequence and a third DNA sequence, wherein the second sequence is complementary to the first sequence, and
      the second surface region comprises
      a third oligonucleotide comprising a fourth DNA sequence and a linking moiety for irreversibly linking the third oligonucleotide to the second surface region, and
         wherein the fourth sequence is complementary to the second sequence, the third sequence, or a combination of at least six continuous nucleotides of the second sequence and six continuous nucleotides of the third sequence; or
   (b) the first surface region comprises
      a first oligonucleotide comprising a first DNA sequence and a linking moiety for irreversibly linking the first oligonucleotide to the first surface region, and
      a second oligonucleotide comprising a second DNA sequence and a third DNA sequence, wherein the second sequence is complementary to the first sequence, and
      the second surface region comprises
      a third oligonucleotide comprising a fourth DNA sequence and a linking moiety for irreversibly linking the third oligonucleotide to the second surface region, and
      a fourth oligonucleotide comprising a fifth DNA sequence and a sixth DNA sequence, wherein the fifth sequence is complementary to the fourth sequence, and
         wherein the second sequence is complementary to the fifth sequence or is complementary to the sixth sequence.

The first or second oligonucleotide may comprise a fluorescent moiety, and the first or second oligonucleotide may comprises a fluorescence quencher. Each of said second DNA sequences may be identical or may not be identical. Each of said plurality of said oligonucleotide complexes may be located in spatially discrete regions on said surface. The linking moiety may comprises an alkyne group including a strained alkyne or an azide group. The fluidic reaction chamber may have a first port to deliver the sample, and optionally a second another port to allow air/fluid exit when sample is introduced into the first port.

Each of the first DNA sequences may have a length of between about 15 and about 80 nucleotides. Each of the third DNA sequences may have a length of between about 5 and about 80 nucleotides. Each of the third DNA sequences may have a length of between about 15 and about 80 nucleotides. Each of the fifth DNA sequences may have a length of between about 5 and about 20 nucleotides. Each of the fourth DNA sequences may have a length of between about 5 and about 80 nucleotides. Each of the sixth DNA sequences may have a length of between about 5 and about 80 nucleotides.

The fluidic reaction chamber may be circular, oval, square, triangular, rectangular, hexagonal, octagonal, rhomboid or trapezoid, or annular as defined herein. The fluidic reaction chamber may not be at a uniform temperature, and the warmest region of the reaction chamber may be at least 10 °C higher than the coldest region of the reaction chamber. The coldest region of the reaction chamber may be between about 10 °C and about 50 °C. The hottest region of the reaction chamber may be between about 51 °C and about 100 °C. The fluidic reaction chamber may further comprise a fluid disposed within the fluidic reaction chamber, said fluid comprising one or more oligonucleotides and hybridization buffer, and the fluid may further comprise a non-specific nucleic acid staining dye.

Yet an additional embodiment comprises a method of amplifying a target nucleic acid comprising (a) providing a fluidic reaction chamber according to claim 1 appended below , wherein said fluidic reaction chamber is in operable relationship to a first and a second heat source, wherein said first and second heat sources are capable of applying differing first and a second heat levels to said annular chamber, wherein said first and second heat levels are not the same; (b) introducing into said fluidic reaction chamber a fluid comprising a target nucleic acid sequence; and (c) applying first and second heat levels to said fluidic reaction chamber.

The method may further comprise detecting amplification of said target nucleic acid. The first or second oligonucleotide may comprise a fluorescent moiety, and the first or second oligonucleotide may comprise a fluorescence quencher. Each of said second DNA sequences may be identical or may not be identical. Each of said plurality of said oligonucleotide complexes are located in spatially discrete regions on said surface. The linking moiety may comprise an alkyne group including a strained alkyne or an azide group.

Each of the first DNA sequences may have a length of between about 15 and about 80 nucleotides. Each of the third DNA sequences may have a length of between about 5 and about 80 nucleotides. Each of the third DNA sequences may have a length of between about 15 and about 80 nucleotides. Each of the fifth DNA sequences may have a length of between about 5 and about 20 nucleotides. Each of the fourth DNA sequences may have a length of between about 5 and about 80 nucleotides. Each of the sixth DNA sequences may have a length of between about 5 and about 80 nucleotides.

The fluidic reaction chamber may be circular, oval, square, rectangular, triangular, hexagonal, octagonal, rhomboid or trapezoid, or annular as defined herein. The fluidic reaction chamber may not be at a uniform temperature, and the warmest region of the reaction chamber may be at least 10 °C higher than the coldest region of the reaction chamber. The coldest region of the reaction chamber may be between about 10 °C and about 50 °C. The hottest region of the reaction chamber may be between about 51 °C and about 100 °C. The fluid further comprises a non-specific nucleic acid staining dye.

Another embodiment comprises a system which is not claimed, comprising a reaction chamber, comprising
(a) a first region and second region, wherein a first oligonucleotide comprising a first nucleotide sequence is functionalized to the first surface region and a second oligonucleotide comprising a second nucleotide sequence is functionalized to the second surface region, and wherein the first nucleotide sequence and the second nucleotide sequence are not identical; (b) a buffer solution amenable for DNA hybridization at a non-uniform temperature, wherein the buffer solution contacts the first surface region and the second surface region; (c) a third oligonucleotide comprising a third nucleotide sequence, wherein the third oligonucleotide is hybridized to the first oligonucleotide; and (d) a first temperature zone and a second temperature zone, wherein the first temperature zone has a temperature at least 10 °C greater than a temperature of the second temperature zone.

The first surface region may be located on a first surface and the second surface region is located on the first surface. The first surface region may be located on a first surface and the second surface region is located on a second surface, wherein the first surface and the second surface are different surfaces. The first nucleotide sequence may comprise a first nucleotide region that is not complementary to the third nucleotide sequence. The third nucleotide sequence may comprise a second nucleotide region that is not complementary to first nucleotide sequence.

The buffer solution may comprise at least 60% by mass water and a cation at a concentration of at least 1 mM. The length of the first oligonucleotide and the length of the second oligonucleotide may be between 5 nucleotides and 20,000 nucleotides. The first oligonucleotide and the length of the second nucleotide may be between 5 nucleotides and 200 nucleotides. The length of the third oligonucleotide may be between 5 nucleotides and 20,000 nucleotides. The first oligonucleotide, the second oligonucleotide and the third oligonucleotide may be identical or differently and may comprise a nucleic acid selected from the group consisting of DNA, RNA, a nucleotide analog, and any combination thereof.

The nucleotide analog may be selected from the group consisting of LNA, PNA a morpholino-oligonucleotide, and any combination thereof. At least one of the first oligonucleotide, the second oligonucleotide and the third oligonucleotide may be functionalized with a chemical moiety, wherein the chemical moiety allows detection of oligonucleotides. The chemical moiety may be selected from the group consisting of TAMRA, ROX, HEX, an organic fluorophore, a quantum dot, a nanoparticle, methylene blue, an electrochemically active molecule, and any combination thereof.

The buffer solution may comprise a detectable molecule, wherein the detectable molecule exhibits a different unit signal when non- irreversibly bound to an oligonucleotide than when free in solution, such as detectable molecule selected from the group consisting of a SybrGreen dye, a Syto dye, and a EvaGreen dye. The first surface and the second surface may be identical or differently selected from the group consisting of glass, quartz, plastic, a polymer, metal, composite material, and surface self-assembled monolayers. The polymer may be PDMS.

The first surface region may comprise a temperature that is 10 °C below a maximum temperature of the buffer solution, and wherein the second surface region may comprise a temperature that is 10 °C below the maximum temperature of the buffer solution. The system may further comprise at least one heating/cooling element in contact with the first surface region and the second surface region. The at least one heating/cooling element may be selected from the group consisting of a hot plate, a heating fan, an IR-heater, and a water bath. The hot plate may be selected from the group consisting of a thermo-resistive heater and a Peltier element. The system may further comprise an enzyme that modifies nucleic acids in a template-directed manner, such as where the enzyme facilitates template-directed extension of a nucleic acid template.

A method for enzyme-free amplification and detection of a nucleic acid target, comprising (a) contacting a sample with a composition in a reaction chamber, wherein the composition comprises:
a buffer solution amenable for DNA hybridization;
a first surface region and a second surface region, wherein the buffer solution contacts the first surface region and the second surface region;
a first oligonucleotide comprising a first nucleotide sequence, wherein the first oligonucleotide is functionalized to the first surface region;
a second oligonucleotide comprising a second nucleotide sequence, wherein the second oligonucleotide is functionalized to the second surface region; and
a third oligonucleotide comprising a third nucleotide sequence, wherein the third oligonucleotide is hybridized to the first oligonucleotide,
wherein the first nucleotide sequence and the second nucleotide sequence are not identical, and wherein the third nucleotide comprises a first nucleotide region that is not complementary to the first nucleotide sequence;
(b) heating differentially a portion of the reaction chamber, wherein a maximum temperature of a region of the chamber is at least 10 °C higher than a minimum temperature of the chamber; and (c) detecting potential amplification.

The reaction chamber may comprise at least one material selected from the group consisting of glass, quartz, plastic, a polymer, metal, and any combination thereof. The polymer may be PDMS. The maximum temperature of a region of the chamber may be between 60 °C and 100 °C. The minimum temperature of the chamber may be between 20 °C and 60 °C. The first surface region and the second surface region may be not heated to within 10 °C of the maximum temperature of a region of the chamber.

The composition may be localized in the reaction chamber, and wherein the first surface region is located on first surface and the second surface region is located the first surface. The composition may be localized in the reaction chamber, wherein the first surface region may be located on a first surface and the second surface region may be located on a second surface, and wherein the first surface and the second surface are different surfaces. Detecting potential amplification may further comprise optical detection of fluorescence changes through a detection device selected from the group consisting of a photodiode, a photomultiplier tube, a fluorescence microscope, a CCD camera, and any other optical detection device. Detecting potential amplification may further comprise electrochemical detection through an electrochemical potentiostat/galvanostat. Detecting potential amplification may further comprise measuring the mass of the first surface region using quartz crystal microbalance technique.

In still a further embodiment, there is provided a method for enzyme-dependent amplification and detection of a nucleic acid target, comprising (a) contacting a sample with a composition in a reaction chamber, wherein the composition comprises:
a buffer solution amenable for DNA hybridization;
a surface region, wherein the buffer solution contacts the surface region;
a first oligonucleotide comprising a first nucleotide sequence, wherein the first oligonucleotide is functionalized to the surface region;
a second oligonucleotide comprising a second nucleotide sequence; and an enzyme that modifies nucleic acids in a template-directed manner, wherein the second nucleotid sequence comprises a nucleotide region that is not complementary to the first nucleotide sequence;
(b) heating differentially a portion of the reaction chamber, wherein a maximum temperature of a region of the chamber is at least 10 °C higher than a minimum temperature of the chamber; and (c) detecting potential amplification.

The first oligonucleotide may comprise a second nucleotide region that is not complementary to the second nucleotide sequence. The enzyme may be selected from the group consisting of a DNA polymerase, a RNA polymerase, a reverse transcriptase, an endonuclease, and an exonuclease. The reaction chamber may comprise at least one material selected from the group consisting of glass, quartz, plastic, a polymer, metal, and any combination thereof. The polymer may be PDMS.

The maximum temperature of a region of the chamber may be between 80 °C and 100 °C. The minimum temperature of the chamber may be between 20 °C and 80 °C. The surface region may not be heated to within 10 °C of the maximum temperature of a region of the chamber. Detecting potential amplification may further comprise optical detection of fluorescence changes through a detection device selected from the group consisting of a photodiode, a photomultiplier tube, a fluorescence microscope, a CCD camera, and any other optical detection device. Detecting potential amplification may further comprise electrochemical detection through an electrochemical potentiostat/galvanostat. Detecting potential amplification may further comprise measuring the mass of the first surface region using quartz crystal microbalance technique.

In one embodiment of the present disclosure, surface oligonucleotide functionalization can be performed by various chemical and physical methods including but not limited to covalent immobilization, electrostatic interaction or non-covalent immobilization such as biotin-avidin (or their analogs).

In one embodiment of the present disclosure, the detection target molecule may be single-stranded DNA, double-stranded DNA, RNA or their mixtures.

In one embodiment of the present disclosure, amplicon detection and reaction monitoring is through methods including but not limited to: fluorescence (including surface plasmon resonance, SPR), UV absorbance, electrochemical detection (including pH change and charge transfer), quartz crystal microbalance (QCM)

In one embodiment of the present disclosure, the proposed approaches can be used for distinguishing nucleic acid sequence variants, including single nucleotide variants (SNVs) that may be indicative of drug resistance or disease prognosis.

In one embodiment of the present disclosure, the proposed approaches can be used for quantitation of one, several, or many target molecules in specific biological samples.

As used herein, the term "annular" may be used to reference the shape of the chamber, as discussed above, and may have its normal meaning of round, oval or discoid. However, annular may also be interpreted in this context to have other regular or irregular shapes so long as the chamber constitutes a continuous circuit with no end and no beginning.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, for the method being employed to determine the value, or that exists among the study subjects. Such an inherent variation may be a variation of ±10% of the stated value.

Throughout this application, the term "irreversible linking" is used to indicate a chemical interaction that is stable under usual circumstances of the intended application. Irreversible linking in some embodiments can refer to covalent attachment such as by azide-alkyne click chemistry, and in other embodiments can refer to biotin-avidin interactions or other non-covalent long-lived interactions.

Throughout this application, the term "PCR buffer" is used to indicate an aqueous solution with salinity and chemical composition compatible with DNA amplification by a DNA polymerase via the polymerase chain reaction (PCR). The buffer may be used in conjunction with the DNA polymerase itself, primers and/or dNTPS.

Throughput this application, the term "hybridization buffer" is used to indicate an aqueous solution with salinity and chemical composition compatible with DNA hybridization and formation of stable DNA duplexes by complementary DNA oligonucleotides. All PCR buffers can be considered hybridization buffers, but not vice versa.

Other objects, features and advantages of the present invention will become apparent from the following detailed description. It should be understood, however, that the detailed description and the specific examples, while indicating preferred embodiments of the invention, are given by way of illustration only, since various changes and modifications within the scope of the invention will become apparent to those skilled in the art from this detailed description. The scope of the invention is defined in the appended claims.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.

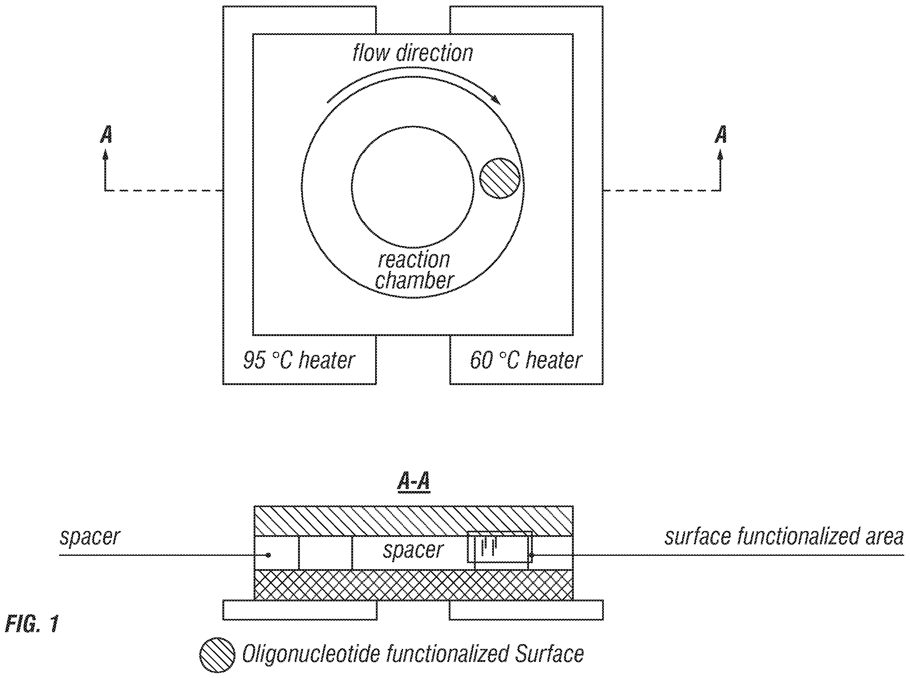

**Figure** 1 shows a schematic of an embodiment of the fluidic chip vertically mounted on heaters at differential temperatures. The fluidic chip comprises a reaction chamber with a surface for functionalizing DNA oligonucleotides. In some embodiments, this chip is used for performing multiplex PCR-based detection of nucleic acids.

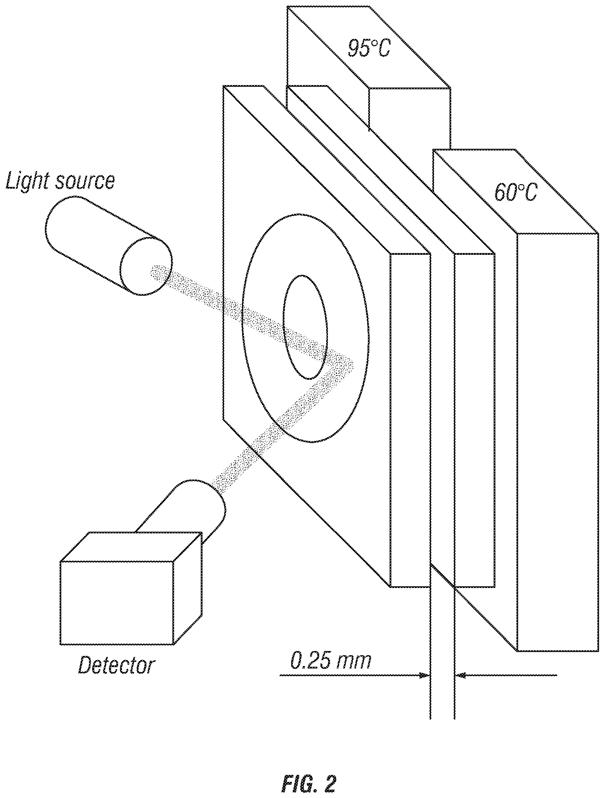

**Figure 2** shows an embodiment of the system, in which the fluidic chip is mounted vertically on the heaters at differential temperatures. A horizontally mounted light source excites fluorescent moieties on DNA functionalized to the chip; fluorescence signal is quantitated via the shown detector.

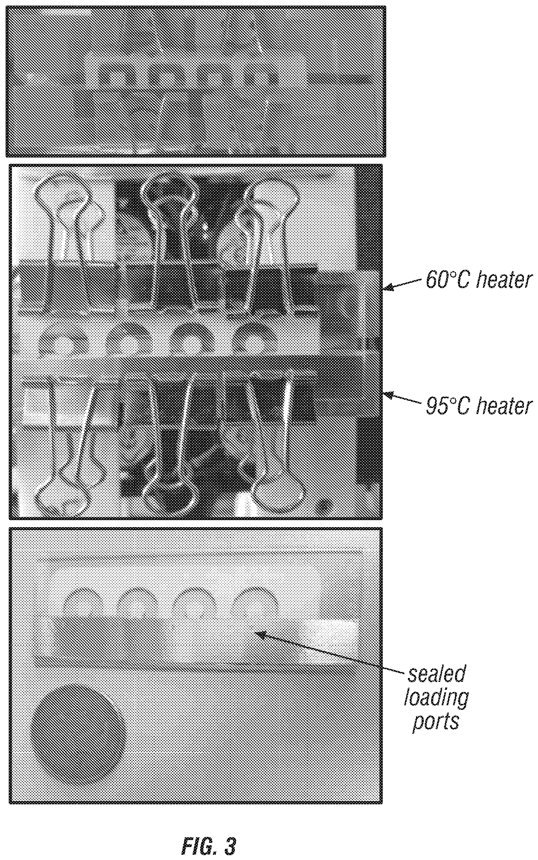

**Figure 3** shows camera pictures of the fluidic chip (left), the chip mounted on heaters (middle) and the chip with rectangular-shaped chambers mounted on the heaters (right).

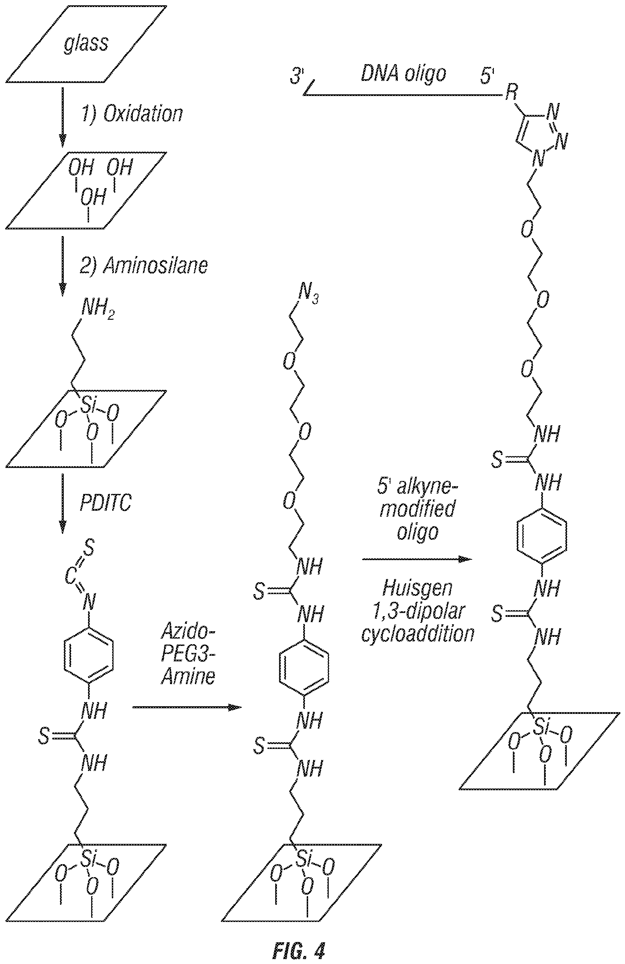

**Figure 4** shows an example of the chemical approach for functionalization of the glass slide with DNA oligonucleotide to form part of the detection probe. The process is a two-step reaction of the PDITC (p-phenylene-diisothiocyanate) activated glass with an azido-PEG-amine (11-azido-3,6,9-trioxaundecan-1-amine) followed by a conjugation with an alkyne-functionalized oligonucleotide via copper-catalyzed alkyne-azide cyclo-addition that results in an oligonucleotide attachment through a hydrophilic PEG linker. Hydrophilicity of the functionalized surface prevents non-specific absorption of the reaction components such as target DNA, primers and enzymes. Copper-free reaction of the azide-functionalized surface with oligonucleotides modified with strained cycloalkynes also results in stable and highly specific covalent attachment of the oligonucleotides to the surface.

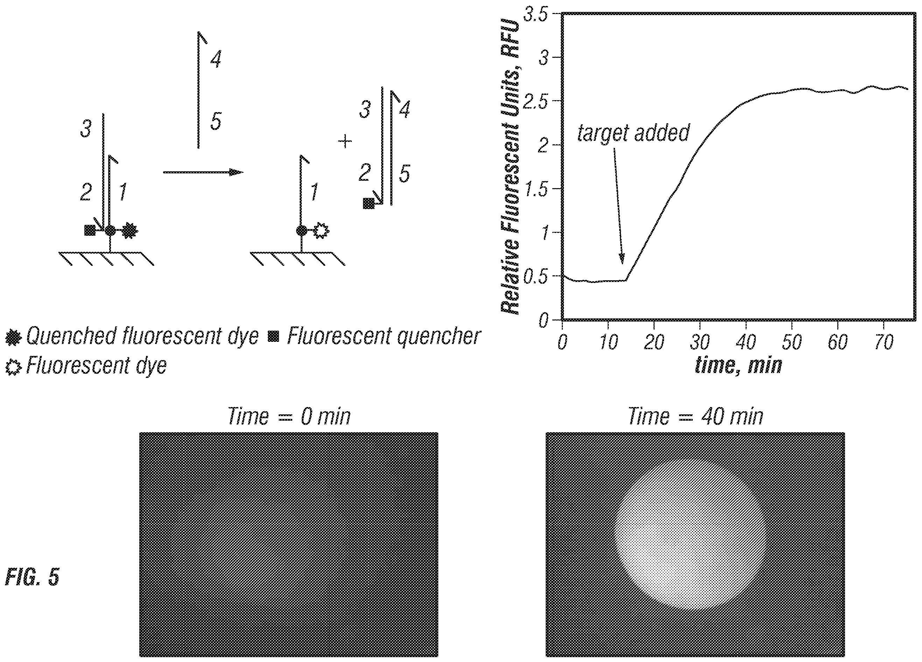

**Figure 5** shows an example of the functionalized probe structure. The oligonucleotide functionalized to the surface is labeled with a fluorophore and the oligonucleotide hybridized to the surface-functionalized probe is labeled with a quencher. When a detection target displaces the quencher-labeled oligonucleotide from the surface, the fluorescence intensity of the surface increases. Bottom panels show fluorescence microscopy images before and after target introduction; the spot diameter here is 1 mm. Oligonucleotides were as follows:
   Alk-TMR-1 /5Hexynyl/tttt/i6-TAMN/tggatgctgaatacttgtgataataca (SEQ ID NO: 1)
   32-RQ ccgtagaggtgtattatcacaagtattcagcatcca/3IAbRQSp/ (SEQ ID NO: 2)
   54 tggatgctgaatacttgtgataatacacctctacgg (SEQ ID NO: 3)

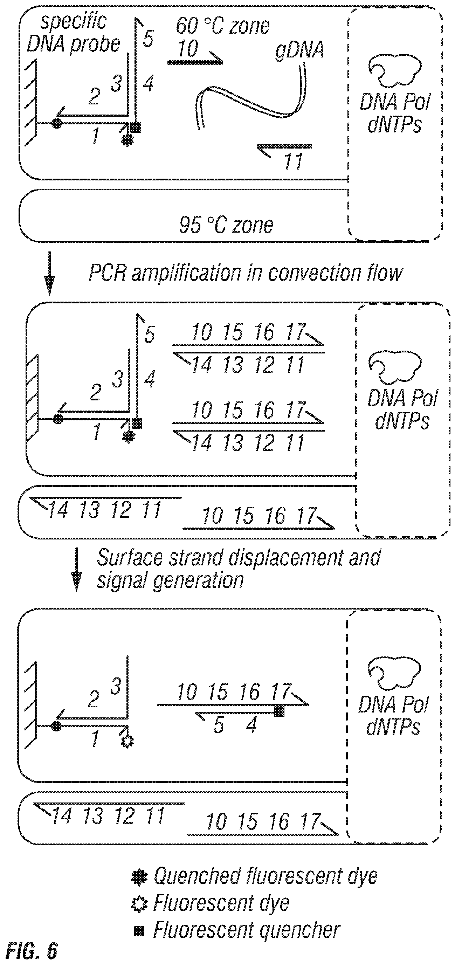

**Figure 6** shows a schematic for polymerase chain reaction (PCR) amplification of a genomic DNA (gDNA) template within the fluidic chip when convection flow is applied; sequence (10) indicates forward primer and sequence (11) indicates reverse primer. The double-stranded amplicon, comprising domains 10-15-16-17 in the forward strand and domains 11-12-13-14 in the reverse strand is denatured in the hot 95 °C zone, and then is carried by convection to the 60 °C zone, where it can displace a quencher-labeled oligonucleotide to generate increased fluorescence in the corresponding spot.

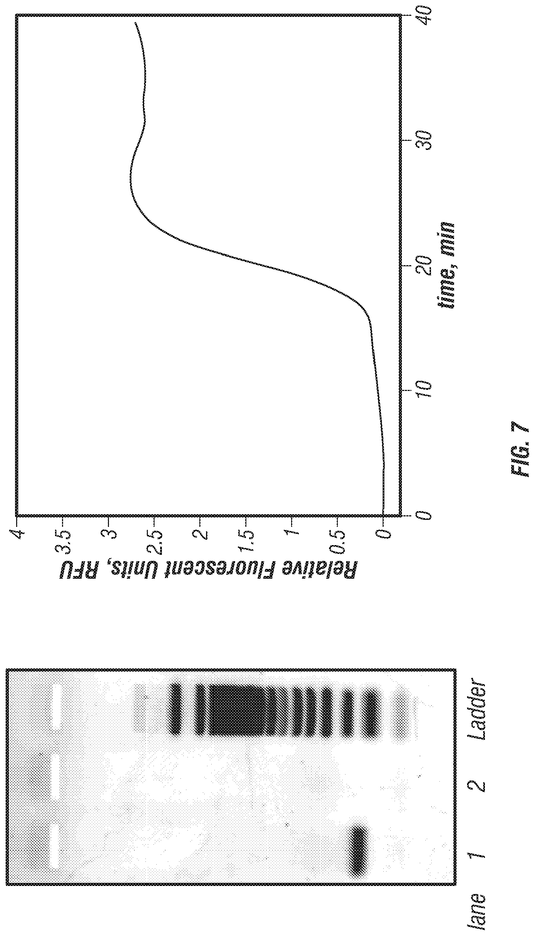

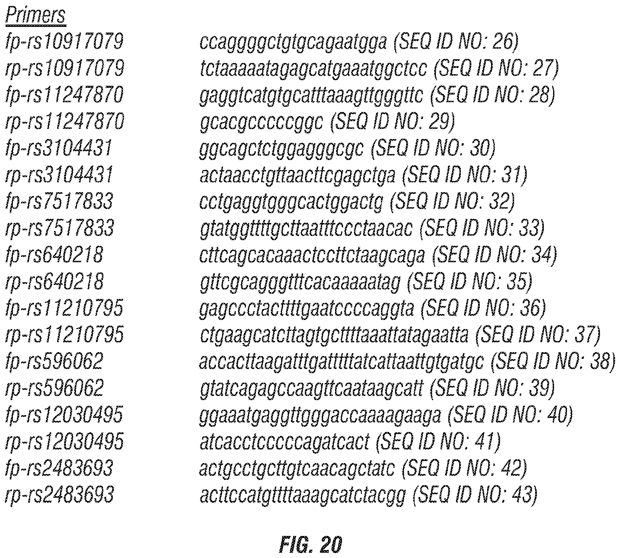

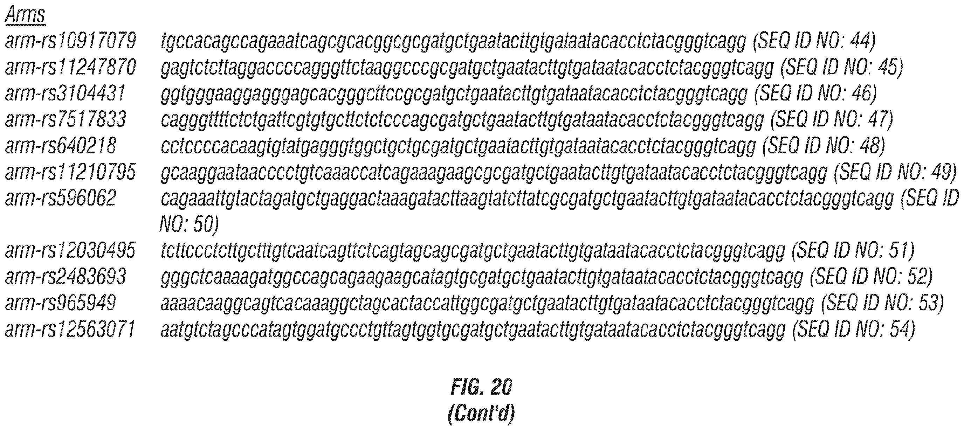

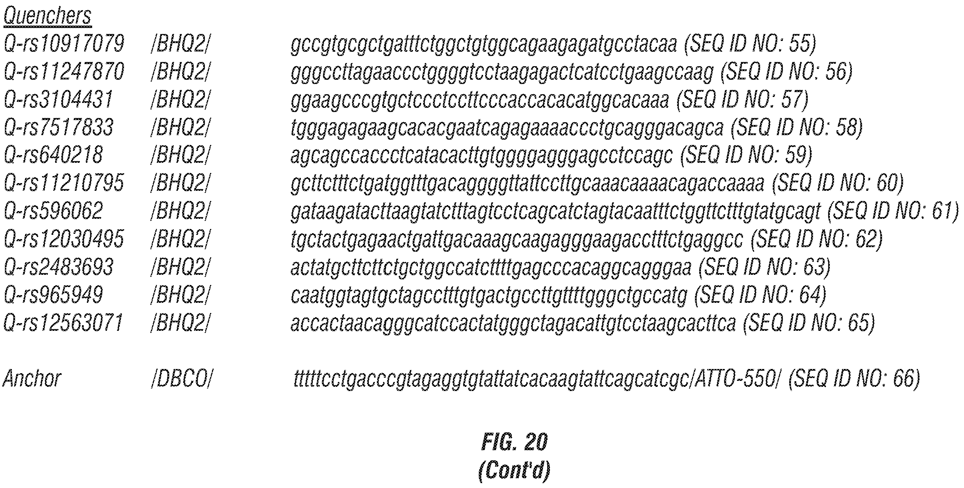

**Figure 7** shows results of PCR amplification within the convection chip. The left panel shows an agarose gel electrophoresis of amplification products. Lane 1 shows the amplification product of 10 ng of NA18562 gDNA template amplified for 30 minutes in the convection chip. Primer concentrations are 600 nM for the forward primer and 200 nM reverse primers. Lane 2 shows a negative control with primers, polymerase, dNTPs, and probe spot, but no gDNA input. The ladder is a 50 bp ladder (New England Biolabs) as a reference. The right panel shows fluorescence time course of the spot intensity through an amplification reaction. The probes and primers for this experiment were designed for target rs7517833 (see Figure 20).

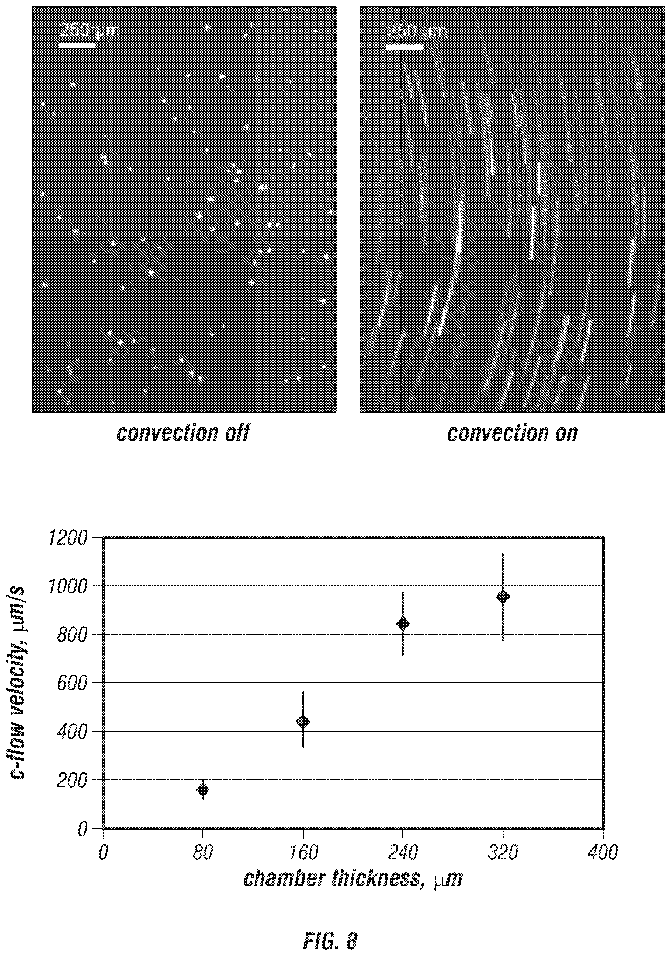

**Figure 8** demonstrates convection flow in the fluidic chip. The top left panel shows a fluorescence image of fluorescent tracking beads in the absence of a temperature gradient across the chip (60 °C for both heaters). The top right panel shows a time-lapse (2 second) fluorescence image of the fluorescence tracking beads when a temperature gradient is applied (95 °C for left heater and 60 °C for right heater). The bottom panel summarizes observed mean convection flow velocity based on chamber thickness.

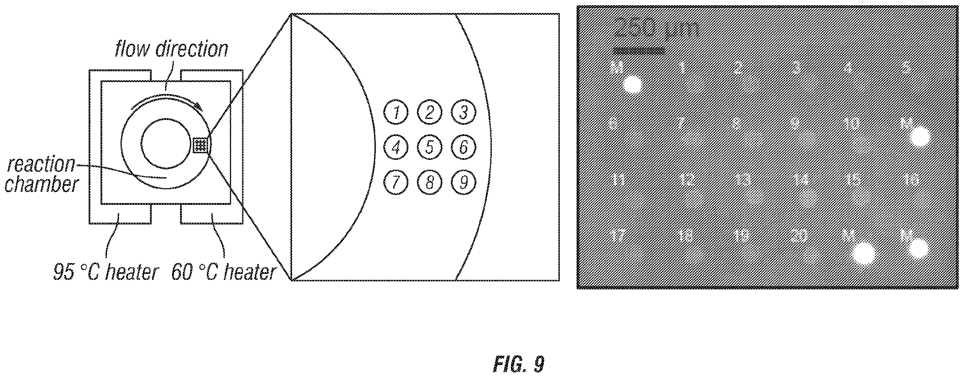

**Figure 9** shows a schematic for an array-based readout of multiple amplicons within the fluidic chip. The right panel shows a fluorescence image of the chip with 24 printed spots. Spots marked M are positive control spots lacking quencher-labeled oligonucleotides. Other spots each are specific to a particular amplicon sequence.

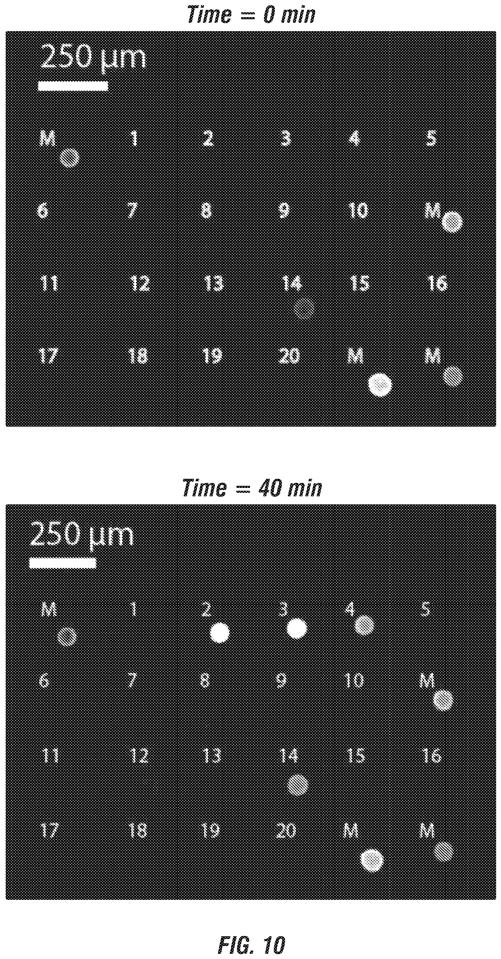

**Figure 10** shows fluorescence images of the probe array area of the fluidic chip before and after convection PCR. Primers that generate amplicons corresponding to the probes at spots 2, 3, and 4 were introduced (600 nM each forward primer, 200 nM each reverse primer), along with 10 ng of gDNA template. High fluorescence of spot 14 is unintentional and may have resulted from poorly functionalized or hybridized DNA probe molecules.

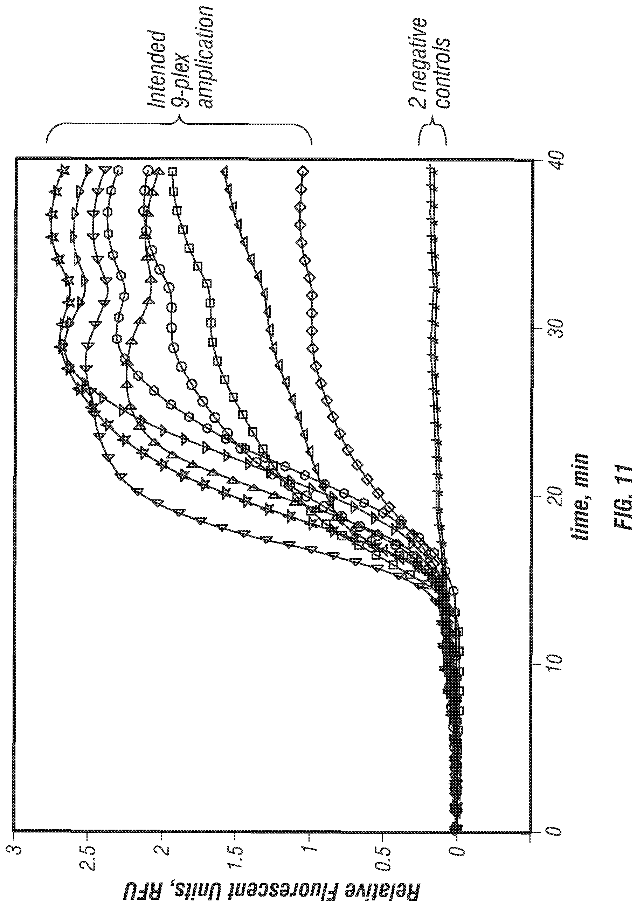

**Figure 11** shows time-course fluorescence for 9-plex PCR amplification in the convection fluidic chip. Each spot's fluorescence intensity was individually quantitated and normalized based on background fluorescence and the fluorescent intensity of the marker spots M. Each forward primer concentration is 200 nM and each reverse primer concentration is 100 nM, and gDNA input is 10 ng.

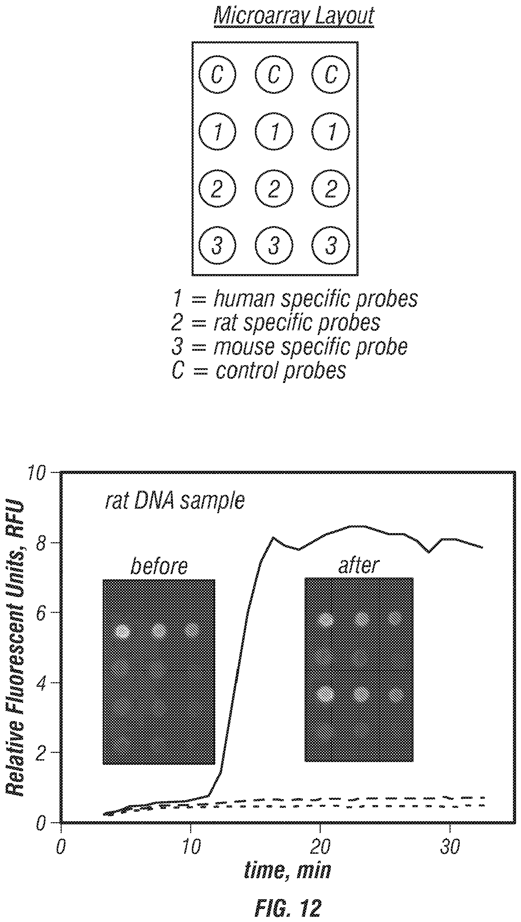

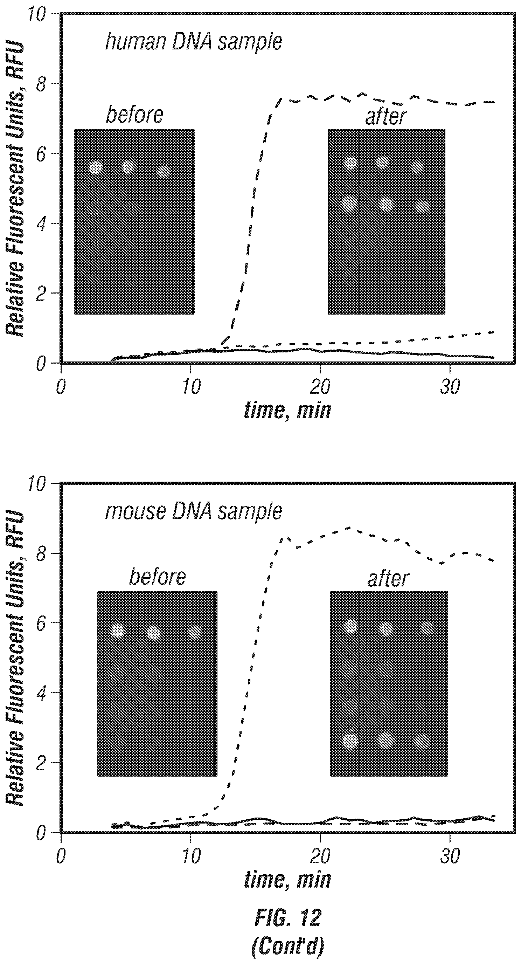

**Figure 12** shows 3-plex PCR amplification in the convection fluidic chip corresponding to primers for human, mouse, and rat DNA. Here, all spots in a row have the same sequence identity and report on the same amplicon. The top row are positive control probes. In the reaction chamber was 600 nM each forward primer, 200 nM each reverse primer, and 10 ng of gDNA template. Sequences used in the experiment were as follows:
   Primers
      h_ppia_fp gttaacagattggaggtagtagcatttt (SEQ ID NO: 4)
      h_ppia_rp tctatcaccaccccccaact (SEQ ID NO: 5)
      r_b2m_fp caggtattttggggtatgattatggtt (SEQ ID NO: 6)
      r_b2m_rp ccaacagaatttaccaggaaacaca (SEQ ID NO: 7)
      m_gadph_fp caatacggccaaatctgaaagacaa (SEQ ID NO: 8)
      m_gadph_rp ctgcaggttctccacacctat (SEQ ID NO: 9)
   Arms
      h_ppia_arm
         agcagtgcttgctgttccttagaattttgccttgtgcgatgctgaatacttgtgataatacacctctacgggtcagg (SEQ ID NO: 10)
      r_b2m_arm
         ctggttcttactgcagggcgtgggaggagcgcgatgctgaatacttgtgataatacacctctacgggtcagg (SEQ ID NO: 11)
      m_gadph_arm
         gatagcctggggctcactacagacccatgagggcgatgctgaatacttgtgataatacacctctacgggtcagg (SEQ ID NO: 12)
   Quenchers
      h_ppia_q /5IAbRQ/acaaggcaaaattctaaggaacagcaagcactgctgcacgatcaggggt (SEQ ID NO: 13)
      r_b2m_q /gctcctcccacgccctgcagtaagaaccagaccccagcctttacac (SEQ ID NO: 14)
      m_gadph_q /5IAbRQ/cctcatgggtctgtagtgagccccaggctatctcatgttcttcagagtgga (SEQ ID NO: 15)
   Anchor
      /DBCO/tttttcctgacccgtagaggtgtattatcacaagtattcagcatcgc/ATTO-550/ (SEQ ID NO: 16)

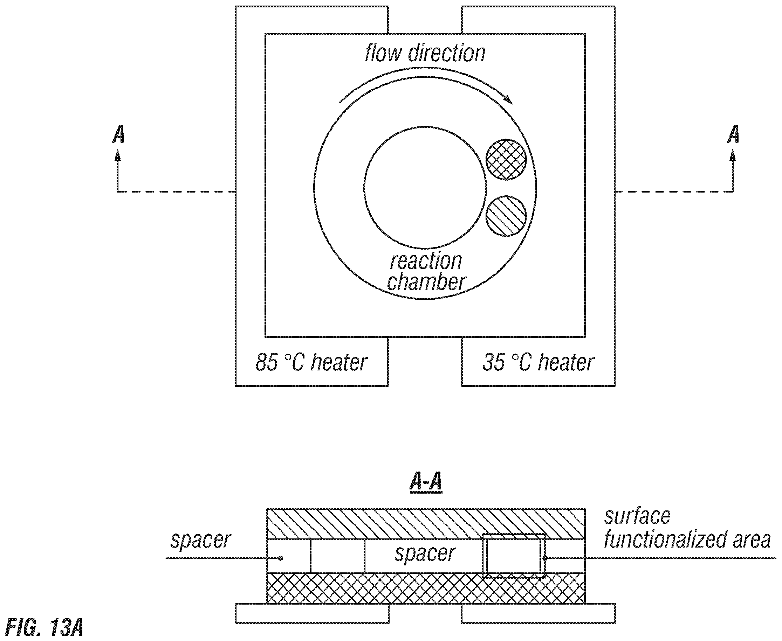

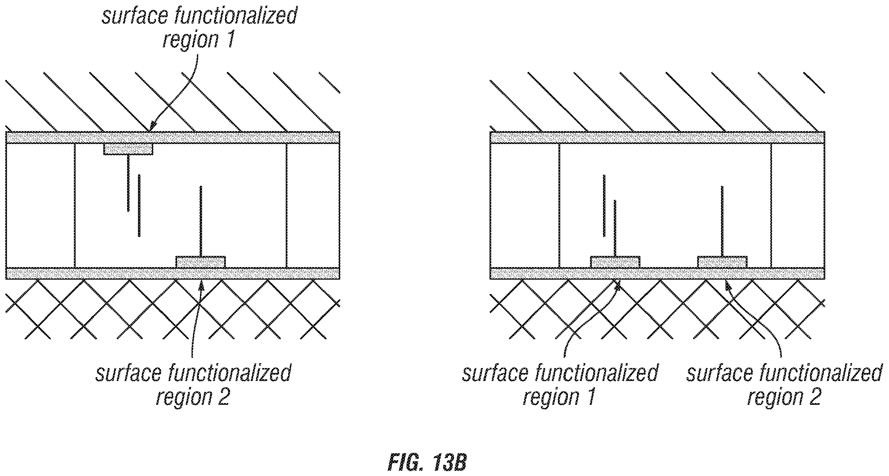

**Figure 13A** shows a schematic of the reaction chamber with two surface regions, each functionalized with different DNA oligonucleotide reagents. Unlike in Figure 1, the oligonucleotide reagents are not independent in sequence, but are rather rationally designed for enzyme-free amplification. **Figure 13B** shows two possible embodiments of the two surface regions: either they are on different surfaces, or on the same surface but distally located to prevent direct interaction.

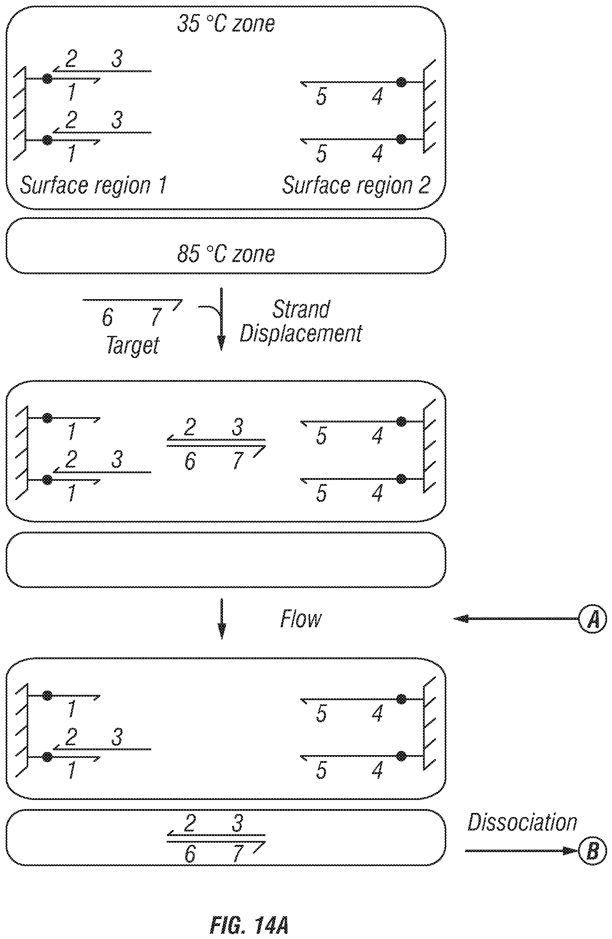

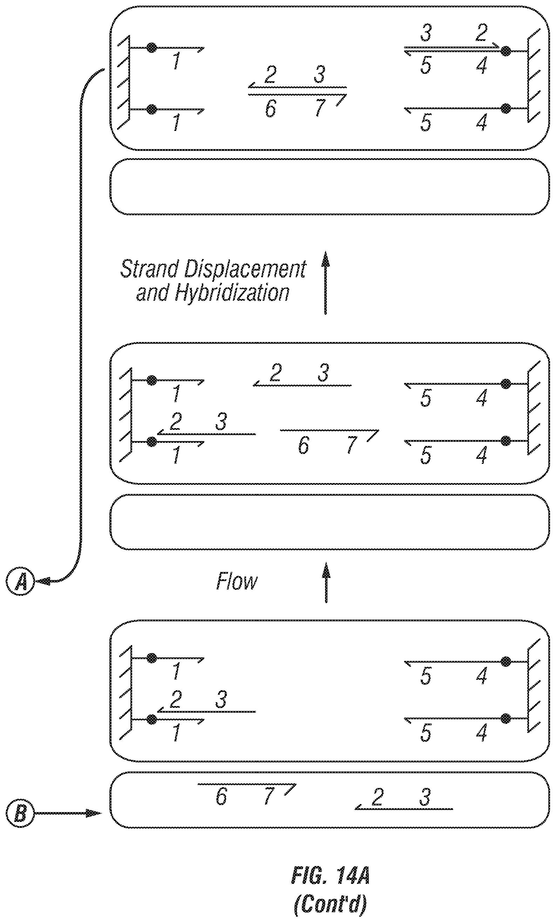

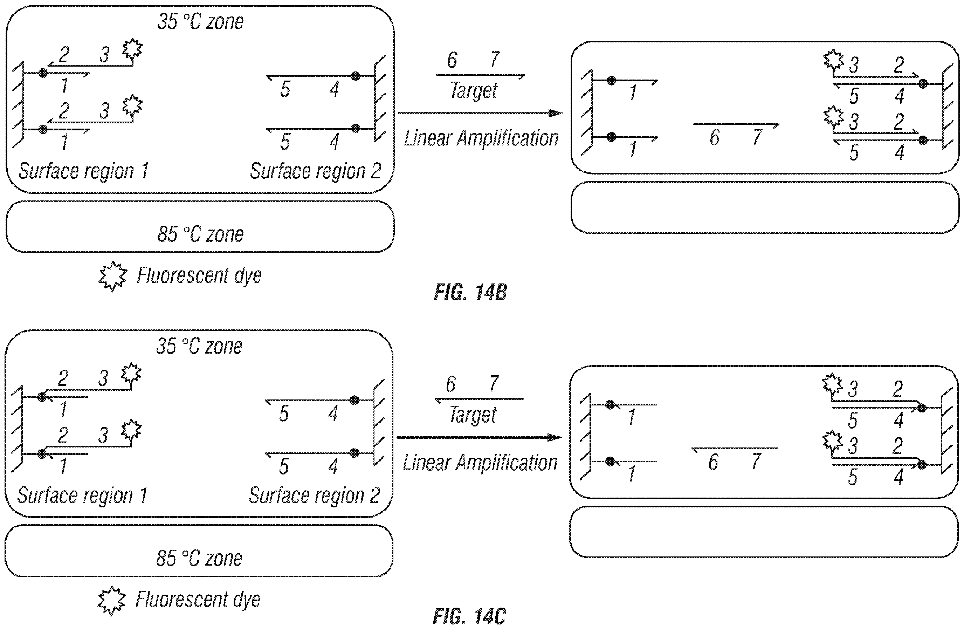

**Figure 14A** shows the mechanism for linear amplification of a target nucleic acid sequence bearing a sixth sequence (6) and a seventh sequence (7). The target nucleic acid sequence catalytically transfers multiple oligonucleotides bearing the second sequence (2) and the third sequence (3) from surface region 1 to surface region 2. Spontaneous dissociation of the double-stranded DNA molecule (23:67) in the hot zone is critical to allow rapid turnover. **Figure 14B** shows the net reaction of the process described in Figure 14A, as well as a fluorescent labeling strategy to allow real-time readout. **Figure 14C** shows an alternative implementation with flipped 5'/3' orientation (the half arrow-head denotes 3' end, as custom in literature).

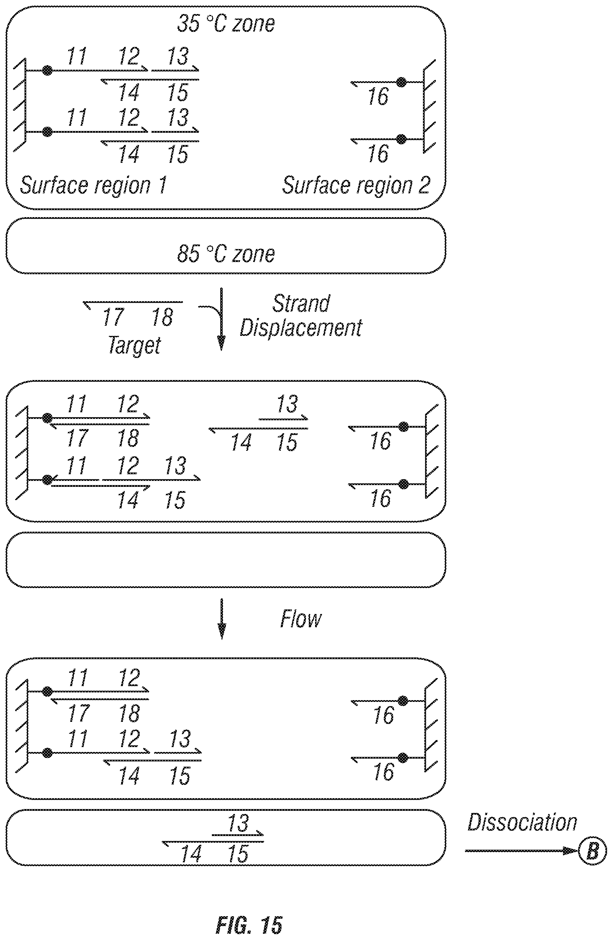

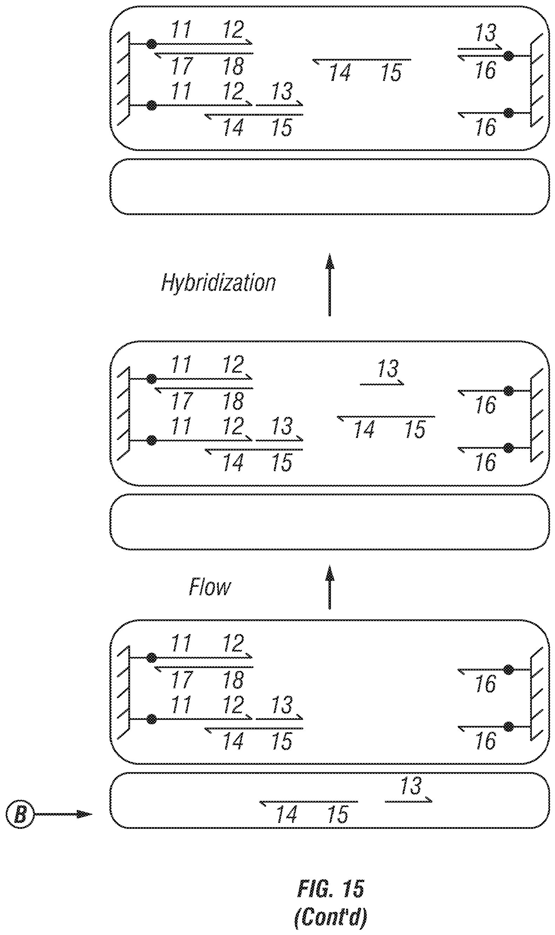

**Figure 15** shows the mechanism for a control experiment in which the target nucleic acid sequence induces a stoichiometric rearrangement of surface-bound oligonucleotides.

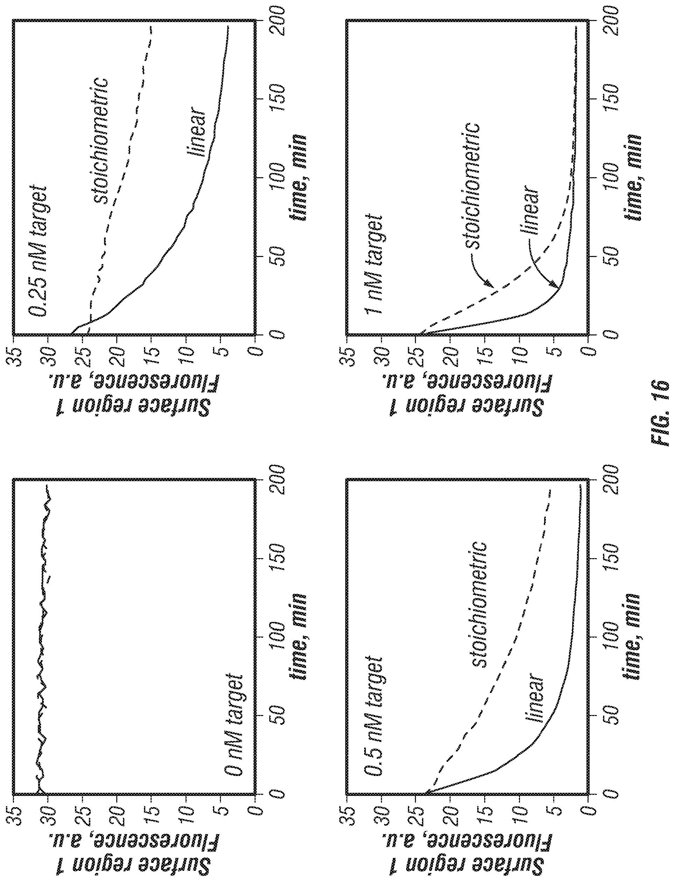

**Figure 16** shows time-course fluorescence of surface region 1 when various concentrations of target nucleic acid are introduced. The fluorescence in the linear amplification chip decreases more quickly than in the stoichiometric conversion chip, supporting the mechanism of enzyme-free DNA amplification.

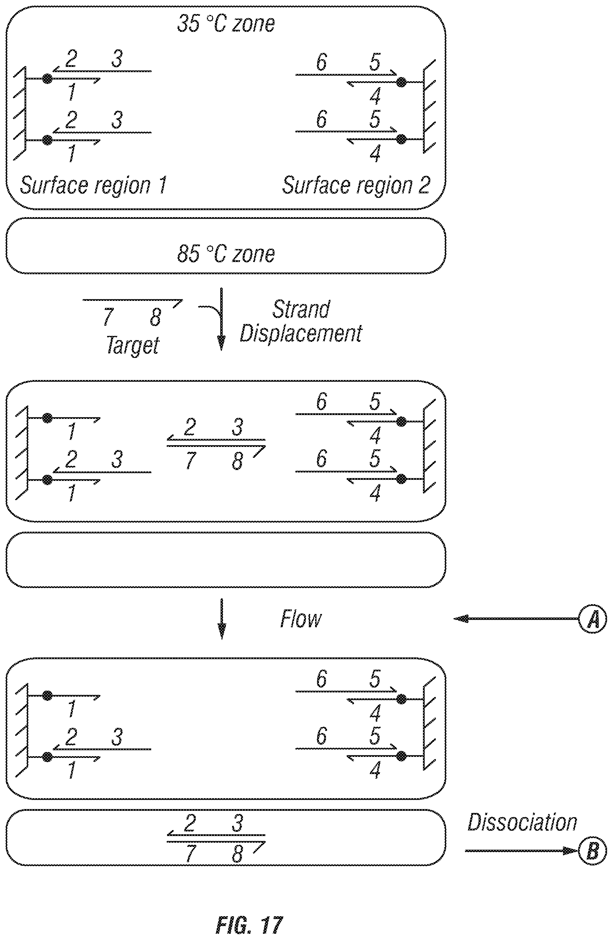

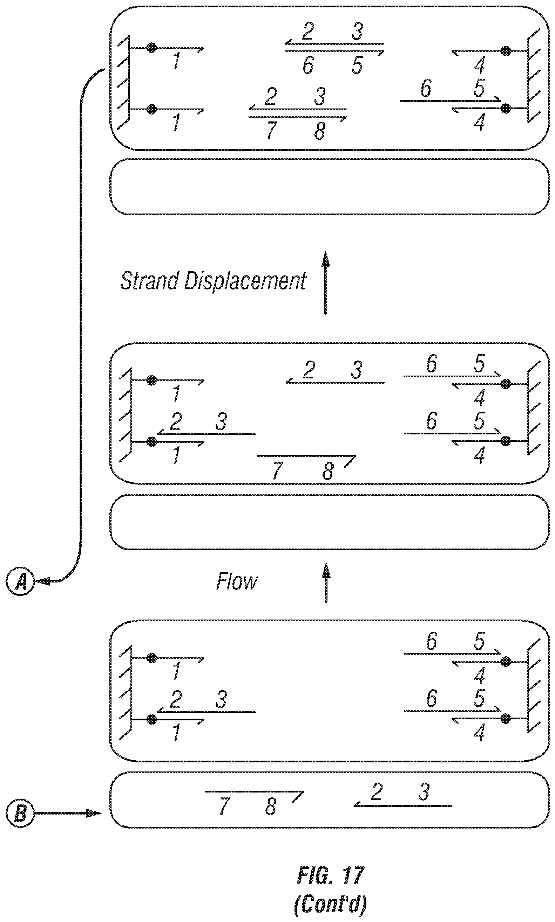

**Figure 17** shows the mechanism for exponential amplification of a target nucleic acid sequence.

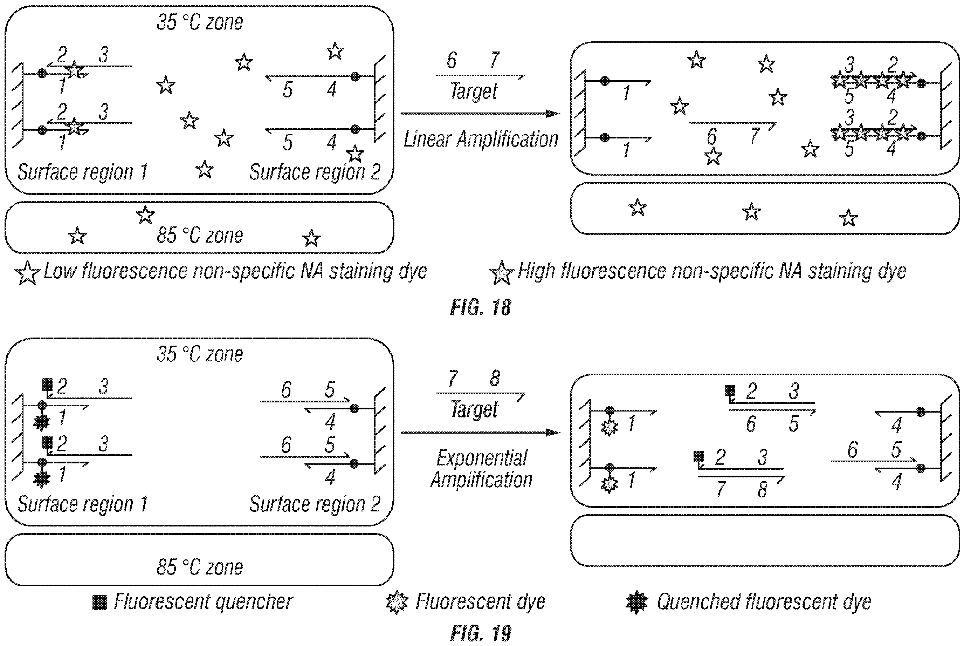

**Figure 18** shows a visual representation of reporting enzyme-free amplification through the use of an intercalating dye, such as SybrGreen or EvaGreen. The fluorescence intensity of surface region 1 will decrease through the course of the reaction, and the fluorescence intensity of surface region 2 will increase.
**Figure 19** shows a visual representation of reporting enzyme-free amplification through the use of a fluorophore-functionalized oligonucleotides. The fluorescence intensity of surface region 1 will increase through the course of the reaction, and the fluorescence intensity of surface region 2 will remain dark through the course of the reaction.
**Figure 20** shows the list of primers and probes (anchor+arm+quencher) used for PCR amplification/detection.

### DETAILED DESCRIPTION

Here, the inventors present devices, systems which are not claimed, and methods for DNA amplification assay. The disclosure employs solid-phase separation of reagents to prevent unintended molecular events resulting in false positives, and uses convection flow circulation to enable spontaneous dissociation of double-stranded amplicons. Three related prior art technologies and their limitations compared to the present invention are described below.

### 1. Convection flow PCR

Liquid, when held at a non-uniform temperature and confined in a volume, will circulate via a process known as Rayleigh-Benard convection flow [¹]. Rayleigh-Benard convection has been used for molecular diagnostics to generate low-cost devices for providing the necessary temperature cycling for PCR (convection flow PCR, cf-PCR) [², U.S. Patent 6,586,233 B2, U.S. Patent 8,735,103 B2, U.S. Patent 8,187,813 B2]. cf-PCR requires only a static temperature gradient maintained with a high of around 95 °C and a low of around 60 °C (annealing/extension temperature), eliminating the need for high energy consumption thermal cycling instruments.

cf-PCR has been demonstrated for both single-plex [³⁻⁵, US patent 8,187,813 B2] and multiplex detection of specific DNA sequences [⁶]; the multiplex approach utilized endpoint electrophoretic results examination. Because cf-PCR lacks the temperature uniformity of traditional qPCR assays, cf-PCR struggles in applications requiring high sequence selectivity, such as applications for detection or profiling of single nucleotide variants (SNV), therefore no SNV specific cf-PCR has yet been shown. Real-time detection of the cf-PCR has been shown solely in solution phase employing unspecific fluorescent dye (SYBR Green I) detection method [⁷]. This approach restricts the cf-PCR from being used in multiplex settings. Likewise, application of sequence specific real-time detection methods such as 5'-nuclease assay chemistry or hybridization probes would allow detecting not more than 5-6 targets simultaneously because of fluorophore spectral overlap. The present disclosure is differentiated from cf-PCR in that the present disclosure offers spatially resolved multiplexed readout without requiring an open-tube step for subsequent analysis. Additionally, in the enzyme-free embodiment of the disclosure, no enzyme is required for amplification.

### 2. Microarrays

Microarray technology is one of the main techniques for multiplexed screening of biological samples. Multiple probe sequences are functionalized to a surface, and the fluorescent signal of a particular spot is taken as the quantitative readout of the corresponding sequence. The technology has been successfully demonstrated for detecting of various types of biological analytes such as DNA, RNA, proteins, carbohydrates and cells [⁸⁻¹²]. Application of the microarray technology has found the most extensive use in the field of nucleic acid testing. Microarray technology has shown application of NA microarrays for whole genome hybridization, *de novo* sequencing, re-sequencing, comparative genomics, transcriptome hybridization or identification of single nucleotide variations [¹³⁻¹⁵]. All aforementioned NA applications require large amounts of NA targets for hybridization, consequently microarrays are typically used as a final readout on PCR amplification products. Microarray readouts are typically slow, requiring overnight hybridization, and also risks amplicon contamination due to the open-tube process.

### 3. Toehold probes and enzyme-free amplification

Toehold-mediated strand displacement reaction [¹⁹⁻²¹] is a process of competitive hybridization that occurs in the absence of enzymes, and is relevant to the present disclosure. Using toehold-mediated strand displacement, enzyme-free amplification of DNA and RNA analyte sequences in homogeneous solutions has been demonstrated [²²⁻²⁷] (U.S. Patent 8,043810 B2, U.S. Patent 8,110,353 B2). The enzyme-free amplification embodiment of the disclosure is different in that thermal convection flow is used to spontaneously dissociate double-stranded amplicons, and surface- functionalization is used to sequester reactive reagents from one another to reduce false positives. Toehold-mediated strand displacement has been applied to surface functionalized DNA oligonucleotides (U.S. Patent 8,630,809 B2) for stoichiometric conversion of target analyte sequences to other sequences. The present disclosure differs in providing amplification of the detection target.

### 4. Applicants' Technology

This disclosure describes reagents and devices for amplification and detection of specific nucleic acid target sequences. The disclosure utilizes solid-phase functionalization and sequestering of oligonucleotide reagents, in order to prevent unintended molecular events that result in false positives, and application of Rayleigh-Benard thermal convection flow for target regeneration and facilitating DNA surface hybridization kinetics (more efficient mixing of the reaction mixture). The Rayleigh-Benard convection flow regime can be realized by placing a reaction chamber, which consists of two 1 mm thick white-water glass microscope slides separated by double- sided sticky tape as a spacer with thickness of 250 µm, between two differentially-controlled hot plates (Figures 1-3) tilted at the defined angle. The shape of the spacer determines the shape of the reaction chamber, and can be modified to alter convection flow speed and trajectory. Glass is selected as the chip material because glass facilitates maintenance of a uniform temperature gradient across the chamber and allows surface functionalization with synthetic oligonucleotides.

The hot plates are set to maintain two different temperatures (cold heater and hot heater, respectively), which cause a temperature gradient across the reaction chamber filled with a liquid reaction mixture. Liquid residing near the hot part of the chamber has a higher temperature and, therefore, is less dense than the liquid residing in the part of the chamber with lower temperature. Such distribution of liquid densities in confined volume results in a difference between buoyancy and gravity forces (near the hot and cold heaters, respectively) that in turn results in organization of circular steady-state convective flow.

All molecules dissolved in the liquid are involved in circulation between temperature zones by being dragged by the convection flow. Traveling along temperature zones the molecules experience periodic temperature variations. For example, a double stranded DNA molecule being placed in the circular convection flow experience multiple cycles of heating and cooling. If the temperature of solution in the hot zone is sufficient to melt the DNA duplex and the temperature of the cold zone is favorable to maintain given nucleic acids in a double-stranded form then the circulation of nucleic acids in this convection flow results in repeatable denaturation and annealing cycles. Observation of the multiple cycles of ds-DNA denaturation and annealing can be performed by various methods, for example using fluorescent microscopy by registering the intensity of the non-specific DNA staining dyes placed in the reaction mixture along with the DNA sample.

A prototype heating instrument consists of two resistive Kapton foil heaters glued to the aluminum plates, and can be simultaneously used to provide differential heating for up to five fluidic chips. Two low-wattage power supplies power the heaters. The proposed amplification system is tolerant to heating element temperature inaccuracies in range of ±2 °C, and does not require precise computer controlled hardware.

### 5. Enzyme-free linear amplification embodiment

The present disclosure represents an enzyme-free amplification of target nucleic acid, in which amplicon concentration increases linearly with time (Figure 14A). Two surface regions are irreversibly functionalized with two DNA oligonucleotides, donor, ***D*** (comprising domain ***1***) and acceptor, ***A*** (comprising domains ***4-5***). The donor oligonucleotide functionalized to the surface region 1 is initially hybridized with a signal strand ***S*** comprising domains ***2-3*,** which are complementary to domains ***4-5*,** in such a way that the single-stranded domain ***2*** is exposed to solution and plays a role of a toehold sequence. The acceptor strand is irreversibly functionalized to surface region 2 and initially represents a single-stranded oligonucleotide. The surface regions 1 and 2 are localized in the temperature zone held at 35 °C (35 °C zone), at which the D-S duplex is designed to be highly stable over the time scale of a detection assay. Target molecule ***T*** (comprising domains ***6-7***) is introduced in the reaction solution and will be transferred by convection flow to the 35 °C zone of the reaction chamber. Target ***T*** binds to the signal ***S*** via domain ***7*** (the "toehold" domain) and displaces domain 3 from surface to the solution via toehold-mediated strand displacement mechanism. Then, Rayleigh-Bernard convection flow carries the duplex to a hot zone of the chamber (held at 85 °C), where the duplex dissociates. The two single-stranded molecules, the target strand ***T*** and the signal strand ***S*** are then transferred back into the chamber's 35 °C zone where strand ***S*** binds to the acceptor oligonucleotide ***A*** functionalized to the surface region 2. At the same time allowing the single-stranded target ***T*** catalytically displaces another signal ***S*** molecule from surface region 1, completing the catalytic cycle. This trigger should proceed continuously until the signal molecules *S* are completely transferred from surface region 1 to surface region 2.

The linear amplification scheme demonstrates the benefits of simultaneously using solid-phase sequestering of oligonucleotide reactants and the temperature-driven convection flow. Immobilization of the oligonucleotide reagents on the different surface regions allows avoiding false positive signal molecule release (spurious amplification) in the absence of the target sequence, while the thermal convection flow, beside spontaneous transport and improved mass transfer, induces target regeneration via melting of the target-signal complex (***T-S***). In contrast, changing the temperature of the entire solution is undesirable because it would lead to spontaneous dissociation of all oligonucleotides from the surface.

Labeling of the signal strand ***S*** with a fluorescent dye represent one approach for real-time monitoring of the linear amplification process. A decrease of the intensity of fluorescence registered form the of the surface region 1, as well as an increase of the intensity of fluorescence registered from the surface region 2 can effectively reflects how the reaction amplification reaction proceeds.

### 6. Stoichiometric detection embodiment

To demonstrate that the enzyme-free amplification method exhibits multiple turnover, the inventors constructed a corresponding stoichiometric detection system using the convection device (Figure 15). The stoichiometric system also includes two surface regions functionalized with a donor, ***Ds*** strand comprising domains ***11-12*** and an acceptor ***As*** strand comprising domain ***16*.** The ***Ds*** strand is hybridized with a signal complex consisting of a bridge oligonucleotide ***Bs*** comprising domains ***14-15*** and a signal oligonucleotide ***Ss*** comprising domain ***13*.** Domain ***14*** of the ***Bs*** strand and the ***As*** strand possess identical sequence. Target molecule ***T*** comprising domains ***17-18*** introduced in the reaction binds to the toehold domain ***11*** of the donor ***Ds*** and then displaces the signal complex into the solution. During this process target ***T*** binds the donor ***Ds*** and is unable to be regenerated in the chamber's hot zone in order to trigger the release of another signal complex from the surfaces region 1. The displaced signal complex is transferred by the convection flow into the 85 °C zone where it dissociates. After the dissociation the signal molecule ***Ss*** is transferred back to the 35 °C zone and is captured by the acceptor oligonucleotide ***As*** functionalized to the surface region 2. Thus, the amount of the captured signal strand ***Ss*** equals the amount of target ***T*** introduced into the system.

Real-time observation of the stoichiometric detection system can also be performed via simple labeling of the signal strand ***Ss*** with a fluorescent dye and registering the change surface region 2 fluorescence intensity. Figure 16 shows the time-based decrease of fluorescence on surface region 1 for the linear amplification and stoichiometric detection systems, given identical initial quantities of detection target. The decrease of fluorescent signal is faster in the linear amplification system than in the linear amplification assay that supports the designed mechanism in which each target molecule is catalytically transferring multiple signal molecules from surface 1 to surface 2.

### 7. Enzyme-free exponential amplification embodiment

Figure 17 shows an embodiment of the present disclosure in which the detection target triggers the release of an amplicon product whose concentration exponentially increases with time, until reagents are consumed (Figure 17). In this system, the two surface regions are functionalized with partially double-stranded oligonucleotide complexes: surface region 1 has a complex consisting of a single domain oligonucleotide 1 irreversibly attached to the surface and hybridized with an oligonucleotide ***S1*** comprising domains ***3-2*** in such a way that the domain ***3*** remains single-stranded and acts as a toehold sequence. The surface region 2 mirrors surface region 1 and has a similar architecture of an oligonucleotide reagent functionalized to the surface: an oligonucleotide comprising domain ***4*** is irreversibly functionalized to the surface, and hybridized with an oligonucleotide ***S2*** comprising domains ***6-5*** wherein domain ***6*** represents a single-stranded toehold. Injection of the target strand ***T,*** comprising domains ***7*** and ***8*** (identical in sequence to domains ***6*** and ***5*,** respectively), into the reaction mixture results in the release of the oligonucleotide ***S1*** from the surface region 1 in the form of double-strand amplicon.

Convection flow carries the duplex to the 85 °C zone where the duplex melts. Now two single-stranded oligonucleotides, the initial target ***T*** and released strand ***S1*** flows back to the 35 °C zone where each of them triggers new release of oligonucleotide species from the surface. In particular, target oligonucleotide ***T*** catalytically releases second strand ***S1*** from the surface region 1, while the initially released strand ***S1*** triggers the release of the strand ***S2*** from the second surface region. Thus, at the end of each convection flow cycle the amount of oligonucleotide species present in solution doubles, resulting in exponential accumulation of the amplicon species in solution phase.

### 8. Alternative detection methods

Figure 14B presents one possible detection mechanism for assaying reaction progress. Alternative readout approaches also utilizing fluorescent microscopy are possible. Non-sequence-specific intercalating nucleic acid staining dyes, such as SybrGreen and Syto dyes, can be used to indicate the total amount of accumulated double-stranded product (Figure 18). Because the acceptor strand ***A*** (domains ***4-5***) immobilized on the surface region 2 is in single-stranded form at the beginning of the reaction, the intercalating dye would have a low affinity to the strand ***A*.** In the course of the reaction more and more strands ***S*** (domains ***2-3***) will hybridize with the surface functionalized strand ***A*** (domains 4-5). The newly formed complexes ***A-S*** now would be efficiently stained with the dye that would cause an increase of the surface region 2 fluorescence. The opposite situation can be potentially observed on the surface region 1.

Application of the FRET-based detection technique is illustrated on the example of exponential amplification (Figure 19). For example, irreversibly labeled with a fluorescent dye surface immobilized strands (domain ***1*** is shown labeled) can be efficiently quenched with a quencher functionalized signal strands (the strand with domains ***2-3*** is shown with a fluorescent quencher) before the target is added. An addition of the target will result in exponential release of the signal strands forms the surface and lighting up of the surface immobilized strands.

### 9. Real-time detection of convection flow PCR

Another application the composition claimed in the present disclosure is that the composition can be used as an efficient mean for surface-based real-time monitoring of an enzymatic nucleic acid amplification process proceeding in the solution. There are no reported examples of real-time monitoring of convection-based PCR using surface functionalized probes.

Figure 6 shows an approach utilizing the claimed composition for real-time monitoring of the PCR reaction performed in the temperature-driven convection flow. Initially a surface region 1 residing in 60 °C zone is functionalized with an ***Anchor*** oligonucleotide comprising domain ***1*** and a fluorescent dye. An ***Arm*** oligonucleotide comprising domains ***3-2*** is hybridized to the ***Anchor*** oligonucleotide through its ***2*** domain. The ***Anchor-Arm*** oligonucleotide complex is in turn hybridized with a ***Quencher*** oligonucleotide comprising domains ***4*** and ***5*** and a fluorescent quencher through its domain ***4*;** domain ***5*** is single-stranded. The reaction solution comprises reagents for enzymatic extension of oligonucleotide primers (domains 10 and 11), such as a DNA polymerase, mixture of deoxynucleotide triphosphates, divalent ions (Mg²⁺). After injection of a target molecule (gDNA), it spontaneously melts in the 95 °C. Then the melted gDNA is transferred into the 60 °C zone by the convection flow where primers anneal to their specific target sequences and extend by DNA polymerase that leads to formation of the double stranded amplicon molecules comprising domains ***10-15-16-17*** in forward strand and ***11-12-13-14*** in reverse strand. The amplicon molecules melt in the 95°C and flow back to the 60°C zone where forward strand ***10-15-16-17*** displaces the ***Quencher*** oligonucleotide form the surface functionalized complex ***Anchor-Arm*** resulting in increasing of the fluorescent signal registered form the surface region 1.

### 10. Simultaneous monitoring of multiple target sequences

The proposed compositions and methods in this disclosure allow for simultaneous monitoring of the amplification (either enzyme-free or enzyme-based) of multiple nucleic acid target sequences. Spatial patterning of different oligonucleotide probes at different surface regions allows an array- or camera-based readout to provide independent information on the amplicon concentrations of each target amplification reaction.

### 11. Exemplary oligonucleotides

The following oligonucleotide sequences are provided by way of example, but not limitation:
Linear amplification system oligonucleotides
   ***1*** /5Hexynyl/TTTTTCCGTAGAGGTGTATTATCACAAGTATT (SEQ ID NO: 17)
   ***2-3*** /56-TAMN/TGGATGCTG-AATACTTGTGATAATACACCTCTACGG (SEQ ID NO: 18)
   ***4-5*** /5Hexynyl/TTTTTCCGTAGAGGTGTATTATCACAAGTATT-CAGCATCCA (SEQ ID NO: 19)
   ***6-7*** CCGTAGAGGTGTATTATCACAAGTATT-CAGCATCCA (SEQ ID NO: 20)
Stoichiometric detection system oligonucleotides
   ***11-12*** /5Hexynyl/TTTTTTGTCAACC-ATCATCGTTCGTACCACAGTGTTCAG (SEQ ID NO: 21)
   ***13*** /56-TAMN/TGGATGCTGAATACTTGTGATAATACACCTCTACGG (SEQ ID NO: 22)
   ***16*** /5Hexynyl/TTTTTCCGTAGAGGTGTATTATCACAAGTATTCAGCATCCA (SEQ ID NO: 24)
   ***17-18*** CTGAACACTGTGGTACGAACGATGAT-GGTTGACA (SEQ ID NO: 25)

### 12. References

The following references provide exemplary procedural or other details supplementary to those set forth herein.
1. Eckert K, Bestehorn M, Thess A. Square cells in surface-tension-driven Benard convection: experiment and theory. J Fluid Mech. 1998; 356:155-197.
2. Krishnan M, Ugaz VM, Burns M a. PCR in a Rayleigh-Benard convection cell. Science. 2002; 298(5594):793. doi:10.1126/science.298.5594.793.
3. Hennig M, Braun D. Convective polymerase chain reaction around micro-immersion heater. Appl Phys Lett. 2005; 87(18):1-3. doi:10.1063/1.2051787.
4. Hsieh Y-F, Yonezawa E, Kuo L-S, Yeh S-H, Chen P-J, Chen P-H. Polymerase chain reaction with phase change as intrinsic thermal control. Appl Phys Lett. 2013; 102(May 2015):2013-2016. doi:10.1063/1.4803442.
5. Priye A, Hassan YA, Ugaz VM. Microscale Chaotic Advection Enables Robust Convective DNA Replication. Anal Chem. 2013; 85(21):10536-10541. doi: 10.1021/ac402611s.
6. Agrawal N, Hassan Y a., Ugaz VM. A pocket-sized convective PCR thermocycler. Angew Chemie - Int Ed. 2007; 46(23):4316-4319. doi:10.1002/anie.200700306.
7. Hsieh YF, Lee DS, Chen PH, et al. A real-time convective PCR machine in a capillary tube instrumented with a CCD-based fluorometer. Sensors Actuators, B Chem. 2013; 183:434-440. doi:10.1016/j.snb.2013.04.003.
8. Chen J, Lozach J, Garcia EW, et al. Highly sensitive and specific microRNA expression profiling using BeadArray technology. Nucleic Acids Res. 2008; 36(14):e87. doi:10.1093/nar/gkn387.
9. Anzai Y, Saito S, Fujimoto K, Kinoshita K, Kato F. Detection and Identification of Species with Bacterial Cells Using a Plastic DNA Array. J Heal Sci. 2008; 54(2):229-234. doi:10.1248/jhs.54.229.
10. Andresen H, Grötzinger C, Zarse K, Kreuzer OJ, Ehrentreich-Förster E, Bier FF. Functional peptide microarrays for specific and sensitive antibody diagnostics. Proteomics. 2006; 6(5):1376-1384. doi:10.1002/pmic.200500343.
11. Chung MY, Kim Y-W, Bae SM, et al. Development of a bead-based multiplex genotyping method for diagnostic characterization of HPV infection. PLoS One. 2012; 7(2):e32259. doi:10.1371/journal.pone.0032259.
12. Dai X, Yang W, Firlar E, Marras S a. E, Libera M. Surface-patterned microgel-tethered molecular beacons. Soft Matter. 2012; 8(11):3067. doi:10.1039/c1sm06702h.
13. Hoheisel JD. Microarray technology: beyond transcript profiling and genotype analysis. Nat Rev Genet. 2006; 7(3):200-210. doi:10.1038/nrg1809.
14. Gitan RS, Shi H, Chen CM, Yan PS, Huang THM. Methylation-specific oligonucleotide microarray: A new potential for high-throughput methylation analysis. Genome Res. 2002; 12(1):158-164. doi:10.1101/gr.202801.
15. Iwasaki H. Accuracy of Genotyping for Single Nucleotide Polymorphisms by a Microarray-Based Single Nucleotide Polymorphism Typing Method Involving Hybridization of Short Allele-Specific Oligonucleotides. DNA Res. 2002; 9(2):59-62. doi:10.1093/dnares/9.2.59.
16. Jenison R, Jaeckel H, Klonoski J, Latorra D, Wiens J. Rapid amplification/detection of nucleic acid targets utilizing a HDA/thin film biosensor. Analyst. 2014; 3763-3769. doi:10.1039/c4an00418c.
17. Chudinov A V., Kolganova N a., Egorov AE, et al. Bridge DNA amplification of cancer-associated genes on cross-linked agarose microbeads. Microchim Acta. 2014; 182(3-4):557-563. doi:10.1007/s00604-014-1357-8.
18. Quan J, Saaem I, Tang N, et al. Parallel on-chip gene synthesis and application to optimization of protein expression. Nat Biotechnol. 2011; 29(5):449-452. doi: 10.103 8/nbt. 1847.
19. Yurke B, Turberfield AJ, Mills AP, Simmel FC, Neumann JL. A DNA-fuelled molecular machine made of DNA. Nature. 2000; 406(6796):605-608. doi:10.1038/35020524.
20. Zhang DY. Cooperative hybridization of oligonucleotides. J Am Chem Soc. 2011; 133(4):1077-1086. doi:10.1021/ja109089q.
21. Zhang DY, Chen SX, Yin P. Optimizing the specificity of nucleic acid hybridization. Nat Chem. 2012;4(3):208-214. doi:10.1038/nchem.1246.
22. Zhang Z, Zeng D, Ma H, et al. A DNA-Origami chip platform for label-free SNP genotyping using toehold-mediated strand displacement. Small. 2010; 6(17):1854-1858. doi:10.1002/smll.201000908.
23. Zhao C, Song Y, Ren J, Qu X. A DNA nanomachine induced by single-walled carbon nanotubes on gold surface. Biomaterials. 2009; 30(9):1739-1745. doi: 10.1016/j.biomaterials.2008.12.034.
24. Zhang X-B, Kong R-M, Lu Y. Metal ion sensors based on DNAzymes and related DNA molecules. Annu Rev Anal Chem (Palo Alto Calif). 2011; 4:105-128. doi: 10.1146/annurev.anchem.111808.073617.
25. Yan H, Zhang X, Shen Z, Seeman NC. A robust DNA mechanical device controlled by hybridization topology. Nature. 2002; 415(6867):62-65. doi:10.1038/415062a.
26. Turberfield A, Mitchell J, Yurke B, Mills A, Blakey M, Simmel F. DNA Fuel for Free-Running Nanomachines. Phys Rev Lett. 2003; 90(11):1-4. doi: 10.1103/PhysRevLett.90.118102.
27. Shlyahovsky B, Li Y, Lioubashevski O, Elbaz J, Willner I. Logic Gates and Antisense DNA Devices Scaffold. ACS Nano. 2009;3(7):1831-1843.
U.S. Patent 6,586,233 B2. Convectively driven PCR thermal-cycling, William J. Benett, James B. Richards, Fred P. Milanovich
U.S. Patent 8,735,103 B2. Natural convection-driven PCR apparatus and method using disposable polymer chip. Kwang-Hyo Chung, Dae-Sik Lee, Hyeon-Bong Pyo, Seon-Hee Park
U.S. Patent 8,187,813 B2. Methods and apparatuses for convective polymerase chain reaction (PCR). Pei-Jer Chen, Ping-Hei Chen, Wen-Pin Chou, Yi-Fan Hsieh, Shiou-Hwei Yeh
U.S. Patent 8,043,810 B2. Analyte detection using autocatalytic chain reactions. John H. Reif, Peng Yin, Thomas H. LaBean, Geetha Shetty, Erik A. Schultes
5. U.S. Patent 8,110,353 B2. Engineered toehold reactions and networks. David Zhang, Andrew J. Turberfield, Erik Winfree
6. U.S. Patent 8,630,809 B2. System and method for propagating information using modified nucleic acids. Daniel J. Kleinbaum
7. U.S. Patent 6,300,070 B1. Solid phase methods for amplifying multiple nucleic acids. T. Christian Boles, Ezra S. Abrams
8. U.S. Patent 7,399,584 B2. Method of comparing a target nucleic acid and a reference nucleic acid. Maureen T. Cronin, Charles Garrett Miyada, Earl A. Hubbell, Mark Chee, Stephen P. A. Fodor, Xiaohua C. Huang, Robert J. Lipshutz, Peter E. Lobban, MacDonald S. Morris, Edward L. Sheldon
9. U.S. Patent 6,642,000 B2. PCR amplification on microarrays of gel immobilized oligonucleotides. Boris Strizhkov, Sergei Tillib, Vladimir Mikhailovich, Andrei Mirzabekov.
10. U.S. Patent 7,846,656 B2. Composition for polymerizing immobilization of biological molecules and method for producing said composition. Andrei Mirzabekov, Alla Rubina, Sergei Pankov.

## Claims

1. A fluidic reaction chamber comprising:
a first surface,
a second surface that does not contact the first surface, wherein said first and second surfaces face each other,
a material contacting the first surface and the second surface and that forms an outer boundary of said reaction chamber, and
a material contacting the first surface and the second surface and that forms an inner boundary of said reaction chamber, wherein
(a) the first surface comprises a plurality oligonucleotide complexes, wherein said oligonucleotide complexes are located in spatially discrete locations of the first surface region and each comprise:
a first oligonucleotide comprising a first DNA sequence and a linking moiety for irreversibly linking the first oligonucleotide to the first surface region, and
a second oligonucleotide comprising a second DNA sequence and a third DNA sequence, wherein the second sequence is complementary to the first sequence, and
the second surface region comprises a plurality oligonucleotide complexes, wherein said oligonucleotide complexes are located in spatially discrete locations of the second surface region and each comprise:
a third oligonucleotide comprising a fourth DNA sequence and a linking moiety for irreversibly linking the third oligonucleotide to the second surface region, and
wherein the fourth sequence is complementary to the second sequence, the third sequence, or a combination of at least six continuous nucleotides of the second sequence and six continuous nucleotides of the third sequence; or
(b) the first surface region comprises a plurality oligonucleotide complexes, wherein said oligonucleotide complexes are located in spatially discrete locations of the first surface region and each comprise:
a first oligonucleotide comprising a first DNA sequence and a linking moiety for irreversibly linking the first oligonucleotide to the first surface region, and
a second oligonucleotide comprising a second DNA sequence and a third DNA sequence, wherein the second sequence is complementary to the first sequence, and
the second surface region comprises a plurality oligonucleotide complexes, wherein said oligonucleotide complexes are located in spatially discrete locations of the second surface region and each comprise:
a third oligonucleotide comprising a fourth DNA sequence and a linking moiety for irreversibly linking the third oligonucleotide to the second surface region, and
a fourth oligonucleotide comprising a fifth DNA sequence and a sixth DNA sequence, wherein the fifth sequence is complementary to the fourth sequence, and
wherein the second sequence is complementary to the fifth sequence or is complementary to the sixth sequence.

2. The fluidic reaction chamber of claim 1, wherein the first or second oligonucleotide comprises a fluorescent moiety.

3. The fluidic reaction chamber of claim 1, wherein the first or second oligonucleotide comprises a fluorescence quencher.

4. The fluidic reaction chamber of any one of claims 1 to 3, wherein each of said second DNA sequences are identical.

5. The fluidic reaction chamber of any one of claims 1 to 3, wherein each of said second DNA sequences are not identical.

6. The fluidic reaction chamber of any preceding claim wherein each of the first DNA sequences have a length of between 15 and 80 nucleotides and/or each of the third DNA sequences have a length of between 5 and 80 nucleotides and/or each of the fifth DNA sequences have a length of between 5 and 20 nucleotides and/or each of the fourth DNA sequences have a length of between 5 and 80 nucleotides and/or each of the sixth DNA sequences have a length of between 5 and 80 nucleotides.

7. The fluidic reaction chamber of any one of claims 1 to 5, wherein each of the first and second oligonucleotides have a length of between 5 and 200 nucleotides; and the third oligonucleotide has a length of between 5 and 20,000 nucleotides.

8. The fluidic reaction chamber of any preceding claim, wherein the materials contacting first and second surfaces and forming the inner and outer boundaries of the chamber have shape of circle, oval, square, rectangle, triangle, hexagon, octagon, rhombus or trapeze, and provide distance between said first and second surfaces of between about 40 microns (40 µm) and about 2 millimeters (2 mm).

9. The fluidic reaction chamber of any preceding claim, wherein the fluidic reaction chamber is not at a uniform temperature, and wherein the warmest region of the reaction chamber is between about 51 °C and about 100 °C, and the coldest region of the reaction chamber is between about 10 °C and about 50 °C.

10. The fluidic reaction chamber of any preceding claim, further comprising a fluid disposed within the fluidic reaction chamber, said fluid comprising one or more oligonucleotides, hybridization buffer, and optionally does not comprise non-specific nucleic acid staining dye.

11. A method of amplifying a target nucleic acid comprising:
(a) providing a fluidic reaction chamber according to any one of claims 1 to 10, wherein said fluidic reaction chamber is in operable relationship to a first and a second heat source, wherein said first and second heat sources are capable of applying differing first and a second heat levels to said annular chamber, wherein said first and second heat levels are not the same;
(b) introducing into said fluidic reaction chamber a fluid comprising a target nucleic acid sequence, wherein the target nucleic acid sequence comprises:
(i) a sixth sequence that is capable of hybridizing to the second sequence of the second oligonucleotide as defined in claim 1(a), and a seventh sequence that is capable of hybridizing to the third sequence of the second oligonucleotide as defined in claim 1(a); or
(ii) a seventh sequence that is capable of hybridizing to the second sequence of the second oligonucleotide as defined in claim 1(b), and an eighth sequence that is capable of hybridizing to the third sequence of the second oligonucleotide as defined in claim 1(b);
and
(c) applying first and second heat levels to said fluidic reaction chamber to cause a temperature gradient across the fluidic reaction chamber, wherein the fluid circulates by convective flow.

12. The method of claim 11, further comprising detecting amplification of said target nucleic acid.

13. The method of claim 11 or 12, wherein the fluid in step (b) further comprises a non-specific nucleic acid staining dye.

## Patentansprüche

1. Fluidische Reaktionskammer, die Folgendes umfasst:
eine erste Oberfläche,
eine zweite Oberfläche, die die erste Oberfläche nicht berührt, wobei die erste und die zweite Oberfläche einander zugewandt sind,
ein Material, das die erste Oberfläche und die zweite Oberfläche berührt und das eine äußere Begrenzung der Reaktionskammer ausbildet, und
ein Material, das die erste Oberfläche und die zweite Oberfläche berührt und das eine innere Begrenzung der Reaktionskammer ausbildet, wobei
(a) die erste Oberfläche mehrere Oligonukleotidkomplexe umfasst, wobei sich die Oligonukleotidkomplexe an räumlich getrennten Orten der ersten Oberflächenregion befinden und jeweils Folgendes umfassen:
ein erstes Oligonukleotid, das eine erste DNA-Sequenz und ein Verbindungsmolekülteil zum irreversiblen Verbinden des ersten Oligonukleotids mit der ersten Oberflächenregion umfasst, und
ein zweites Oligonukleotid, das eine zweite DNA-Sequenz und eine dritte DNA-Sequenz umfasst, wobei die zweite Sequenz komplementär zu der ersten Sequenz ist, und
die zweite Oberflächenregion mehrere Oligonukleotidkomplexe umfasst, wobei sich die Oligonukleotidkomplexe an räumlich getrennten Orten der zweiten Oberflächenregion befinden und jeweils Folgendes umfassen:
ein drittes Oligonukleotid, das eine vierte DNA-Sequenz und ein Verbindungsmolekülteil zum irreversiblen Verbinden des dritten Oligonukleotids mit der zweiten Oberflächenregion umfasst, und
wobei die vierte Sequenz komplementär zu der zweiten Sequenz, der dritten Sequenz oder einer Kombination aus wenigstens sechs fortlaufenden Nukleotiden der zweiten Sequenz und sechs fortlaufenden Nukleotiden der dritten Sequenz ist; oder
(b) die erste Oberflächenregion mehrere Oligonukleotidkomplexe umfasst, wobei sich die Oligonukleotidkomplexe an räumlich getrennten Orten der ersten Oberflächenregion befinden und jeweils Folgendes umfassen:
ein erstes Oligonukleotid, das eine erste DNA-Sequenz und ein Verbindungsmolekülteil zum irreversiblen Verbinden des ersten Oligonukleotids mit der ersten Oberflächenregion umfasst, und
ein zweites Oligonukleotid, das eine zweite DNA-Sequenz und eine dritte DNA-Sequenz umfasst, wobei die zweite Sequenz komplementär zu der ersten Sequenz ist, und
die zweite Oberflächenregion mehrere Oligonukleotidkomplexe umfasst, wobei sich die Oligonukleotidkomplexe an räumlich getrennten Orten der zweiten Oberflächenregion befinden und jeweils Folgendes umfassen:
ein drittes Oligonukleotid, das eine vierte DNA-Sequenz und ein Verbindungsmolekülteil zum irreversiblen Verbinden des dritten Oligonukleotids mit der zweiten Oberflächenregion umfasst, und
ein viertes Oligonukleotid, das eine fünfte DNA-Sequenz und eine sechste DNA-Sequenz umfasst, wobei die fünfte Sequenz komplementär zu der vierten Sequenz ist, und
wobei die zweite Sequenz komplementär zu der fünften Sequenz oder komplementär zu der sechsten Sequenz ist.

2. Fluidische Reaktionskammer nach Anspruch 1, wobei das erste oder das zweite Oligonukleotid ein fluoreszierendes Molekülteil umfasst.

3. Fluidische Reaktionskammer nach Anspruch 1, wobei das erste oder das zweite Oligonukleotid einen Fluoreszenzquencher umfasst.

4. Fluidische Reaktionskammer nach einem der Ansprüche 1 bis 3, wobei jede der zweiten DNA-Sequenzen identisch sind.

5. Fluidische Reaktionskammer nach einem der Ansprüche 1 bis 3, wobei jede der zweiten DNA-Sequenzen nicht identisch ist.

6. Fluidische Reaktionskammer nach einem der vorhergehenden Ansprüche, wobei jede der ersten DNA-Sequenzen eine Länge zwischen 15 und 80 Nukleotiden aufweist und/oder jede der dritten DNA-Sequenzen eine Länge zwischen 5 und 80 Nukleotiden aufweist und/oder jede der fünften DNA-Sequenzen eine Länge zwischen 5 und 20 Nukleotiden aufweist und/oder jede der vierten DNA-Sequenzen eine Länge zwischen 5 und 80 Nukleotiden aufweist und/oder jede der sechsten DNA-Sequenzen eine Länge zwischen 5 und 80 Nukleotiden aufweist.

7. Fluidische Reaktionskammer nach einem der Ansprüche 1 bis 5, wobei jedes der ersten und der zweiten Oligonukleotide eine Länge zwischen 5 und 200 Nukleotiden aufweist; und das dritte Oligonukleotid eine Länge zwischen 5 und 20.000 Nukleotiden aufweist.

8. Fluidische Reaktionskammer nach einem der vorhergehenden Ansprüche, wobei die Materialien, die erste und zweite Oberflächen berühren und die inneren und die äußeren Begrenzungen der Kammer ausbilden, die Form eines Kreises, eines Ovals, eines Quadrats, eines Rechtecks, eines Dreiecks, eines Sechsecks, eines Achtecks, einer Raute oder eines Trapezes aufweisen und einen Abstand zwischen der ersten und der zweiten Oberfläche zwischen etwa 40 Mikrometer (40 µm) und etwa 2 Millimeter (2 mm) bereitstellen.

9. Fluidische Reaktionskammer nach einem der vorhergehenden Ansprüche, wobei die fluidische Reaktionskammer keine gleichmäßige Temperatur aufweist und wobei die wärmste Region der Reaktionskammer zwischen etwa 51 °C und etwa 100 °C liegt und die kälteste Region der Reaktionskammer zwischen etwa 10 °C und etwa 50 °C liegt.

10. Fluidische Reaktionskammer nach einem der vorhergehenden Ansprüche, die ferner ein Fluid umfasst, das innerhalb der fluidischen Reaktionskammer angeordnet ist, wobei das Fluid ein oder mehrere Oligonukleotide, Hybridisierungspuffer und optional keinen unspezifischen Nukleinsäure-Anfärbefarbstoff umfasst.

11. Verfahren zum Amplifizieren einer Zielnukleinsäure, das Folgendes umfasst:
(a) Bereitstellen einer fluidischen Reaktionskammer nach einem der Ansprüche 1 bis 10, wobei die fluidische Reaktionskammer in betriebsfähiger Beziehung zu einer ersten und einer zweiten Wärmequelle steht, wobei die erste und die zweite Wärmequelle in der Lage sind, unterschiedliche erste und zweite Wärmeniveaus zu der ringförmigen Kammer zuzuführen, wobei das erste und das zweite Wärmeniveau nicht gleich sind;
(b) Einführen eines Fluids, das eine Zielnukleinsäuresequenz umfasst, in die fluidischen Reaktionskammer, wobei die Zielnukleinsäuresequenz Folgendes umfasst:
(i) eine sechste Sequenz, die in der Lage ist, mit der zweiten Sequenz des zweiten Oligonukleotids, wie in Anspruch 1(a) definiert, zu hybridisieren, und eine siebte Sequenz, die in der Lage ist, mit der dritten Sequenz des zweiten Oligonukleotids, wie in Anspruch 1(a) definiert, zu hybridisieren; oder
(ii) eine siebte Sequenz, die in der Lage ist, mit der zweiten Sequenz des zweiten Oligonukleotids, wie in Anspruch 1(b) definiert, zu hybridisieren, und eine achte Sequenz, die in der Lage ist, mit der dritten Sequenz des zweiten Oligonukleotids, wie in Anspruch 1(b) definiert, zu hybridisieren;
und
(c) Anwenden erster und zweiter Wärmeniveaus auf die fluidische Reaktionskammer, um einen Temperaturgradienten über die fluidische Reaktionskammer hinweg zu bewirken, wobei das Fluid durch Konvektionsströmung zirkuliert.

12. Verfahren nach Anspruch 11, das ferner ein Nachweisen der Amplifikation der Zielnukleinsäure umfasst.

13. Verfahren nach Anspruch 11 oder 12, wobei das Fluid in Schritt (b) ferner einen unspezifischen Nukleinsäure-Anfärbefarbstoff umfasst.

## Revendications

1. Chambre de réaction fluidique comprenant :
une première surface,
une seconde surface qui n'entre pas en contact avec la première surface, lesdites première et seconde surfaces se faisant face,
un matériau en contact avec la première surface et la seconde surface et qui forme une limite externe de ladite chambre de réaction, et
un matériau en contact avec la première surface et la seconde surface et qui forme une limite interne de ladite chambre de réaction,
(a) la première surface comprenant une pluralité de complexes d'oligonucléotides, lesdits complexes d'oligonucléotides étant situés à des emplacements spatialement distincts de la première région de surface et comprenant chacun :
un premier oligonucléotide comprenant une première séquence d'ADN et une fraction de liaison permettant de lier de manière irréversible le premier oligonucléotide à la première région de surface, et
un deuxième oligonucléotide comprenant une deuxième séquence d'ADN et une troisième séquence d'ADN, la deuxième séquence étant complémentaire à la première séquence, et
la seconde région de surface comprenant une pluralité de complexes d'oligonucléotides, lesdits complexes d'oligonucléotides étant situés à des emplacements spatialement distincts de la seconde région de surface et comprenant chacun :
un troisième oligonucléotide comprenant une quatrième séquence d'ADN et une fraction de liaison permettant de lier de manière irréversible le troisième oligonucléotide à la seconde région de surface, et
la quatrième séquence étant complémentaire à la deuxième séquence, à la troisième séquence, ou à une combinaison d'au moins six nucléotides continus de la deuxième séquence et de six nucléotides continus de la troisième séquence ; ou
(b) la première région de surface comprenant une pluralité de complexes d'oligonucléotides, lesdits complexes d'oligonucléotides étant situés à des emplacements spatialement distincts de la première région de surface et comprenant chacun :
un premier oligonucléotide comprenant une première séquence d'ADN et une fraction de liaison permettant de lier de manière irréversible le premier oligonucléotide à la première région de surface, et
un deuxième oligonucléotide comprenant une deuxième séquence d'ADN et une troisième séquence d'ADN, la deuxième séquence étant complémentaire à la première séquence, et
la seconde région de surface comprenant une pluralité de complexes d'oligonucléotides, lesdits complexes d'oligonucléotides étant situés à des emplacements spatialement distincts de la seconde région de surface et comprenant chacun :
un troisième oligonucléotide comprenant une quatrième séquence d'ADN et une fraction de liaison permettant de lier de manière irréversible le troisième oligonucléotide à la seconde région de surface, et
un quatrième oligonucléotide comprenant une cinquième séquence d'ADN et une sixième séquence d'ADN, la cinquième séquence étant complémentaire à la quatrième séquence, et
la deuxième séquence étant complémentaire à la cinquième séquence ou étant complémentaire à la sixième séquence.

2. Chambre de réaction fluidique selon la revendication 1, dans laquelle le premier ou le deuxième oligonucléotide comprend une fraction fluorescente.

3. Chambre de réaction fluidique selon la revendication 1, dans laquelle le premier ou le deuxième oligonucléotide comprend un extincteur de fluorescence.

4. Chambre de réaction fluidique selon l'une quelconque des revendications 1 à 3, dans laquelle chacune desdites deuxièmes séquences d'ADN est identique.

5. Chambre de réaction fluidique selon l'une quelconque des revendications 1 à 3, dans laquelle chacune desdites deuxièmes séquences d'ADN n'est pas identique.

6. Chambre de réaction fluidique selon l'une quelconque des revendications précédentes, dans laquelle chacune des premières séquences d'ADN a une longueur comprise entre 15 et 80 nucléotides et/ou chacune des troisièmes séquences d'ADN a une longueur comprise entre 5 et 80 nucléotides et/ou chacune des cinquièmes séquences d'ADN a une longueur comprise entre 5 et 20 nucléotides et/ou chacune des quatrièmes séquences d'ADN a une longueur comprise entre 5 et 80 nucléotides et/ou chacune des sixièmes séquences d'ADN a une longueur comprise entre 5 et 80 nucléotides.

7. Chambre de réaction fluidique selon l'une quelconque des revendications 1 à 5, dans laquelle chacun parmi le premier et le deuxième oligonucléotides a une longueur comprise entre 5 et 200 nucléotides ; et le troisième oligonucléotide a une longueur comprise entre 5 et 20 000 nucléotides.

8. Chambre de réaction fluidique selon l'une quelconque des revendications précédentes, dans laquelle les matériaux en contact avec les première et seconde surfaces et formant les limites interne et externe de la chambre ont la forme d'un cercle, d'un ovale, d'un carré, d'un rectangle, d'un triangle, d'un hexagone, d'un octogone, d'un losange ou d'un trapèze, et fournissent une distance entre lesdites première et seconde surfaces comprise entre environ 40 microns (40 µm) et environ 2 millimètres (2 mm).

9. Chambre de réaction fluidique selon l'une quelconque des revendications précédentes, dans laquelle la chambre de réaction fluidique n'est pas à une température uniforme, et dans laquelle la région la plus chaude de la chambre de réaction est comprise entre environ 51 °C et environ 100 °C, et la région la plus froide de la chambre de réaction est comprise entre environ 10 °C et environ 50 °C.

10. Chambre de réaction fluidique selon l'une quelconque des revendications précédentes, comprenant en outre un fluide disposé à l'intérieur de la chambre de réaction fluidique, ledit fluide comprenant un ou plusieurs oligonucléotides, un tampon d'hybridation et éventuellement ne comprenant pas de colorant d'imprégnation d'acide nucléique non spécifique.

11. Procédé permettant l'amplification d'un acide nucléique cible comprenant :
(a) la fourniture d'une chambre de réaction fluidique selon l'une quelconque des revendications 1 à 10, ladite chambre de réaction fluidique étant en relation fonctionnelle avec une première et une seconde sources de chaleur, lesdites première et seconde sources de chaleur étant capables d'appliquer différents premier et second niveaux de chaleur à ladite chambre annulaire, lesdits premier et second niveaux de chaleur n'étant pas les mêmes ;
(b) l'introduction dans ladite chambre de réaction fluidique d'un fluide comprenant une séquence d'acide nucléique cible, la séquence d'acide nucléique cible comprenant :
(i) une sixième séquence qui est capable de s'hybrider à la deuxième séquence du deuxième oligonucléotide tel que défini dans la revendication 1(a), et une septième séquence qui est capable de s'hybrider à la troisième séquence du deuxième oligonucléotide tel que défini dans la revendication 1(a) ; ou
(ii) une septième séquence qui est capable de s'hybrider à la deuxième séquence du deuxième oligonucléotide tel que défini dans la revendication 1(b), et une huitième séquence qui est capable de s'hybrider à la troisième séquence du deuxième oligonucléotide tel que défini dans la revendication 1(b) ;
et
(c) l'application des premier et second niveaux de chaleur à ladite chambre de réaction fluidique pour provoquer un gradient de température à travers la chambre de réaction fluidique, le fluide circulant par écoulement convectif.

12. Procédé selon la revendication 11, comprenant en outre la détection de l'amplification dudit acide nucléique cible.

13. Procédé selon la revendication 11 ou 12, dans lequel le fluide à l'étape (b) comprend en outre un colorant d'imprégnation d'acide nucléique non spécifique.


### SEQUENCE LISTING

<110> William Marsh Rice University
<120> SURFACE-BASED DETECTION OF NUCLEIC ACIDS IN CONVECTION FLOW FLUIDIC DEVICES
<130> RICE.P0022WO
<140> Unknown
   <141> 2017-03-28
<150> US 62/314,909
   <151> 2016-03-29
<160> 66
<170> PatentIn version 3.5
<210> 1
   <211> 31
   <212> DNA
   <213> Artificial sequence
<220>
   <223> Synthetic oligonucleotide
<400> 1
   tttttggatg ctgaatactt gtgataatac a 31
<210> 2
   <211> 36
   <212> DNA
   <213> Artificial sequence
<220>
   <223> Synthetic oligonucleotide
<400> 2
   ccgtagaggt gtattatcac aagtattcag catcca 36
<210> 3
   <211> 36
   <212> DNA
   <213> Artificial sequence
<220>
   <223> Synthetic oligonucleotide
<400> 3
   tggatgctga atacttgtga taatacacct ctacgg 36
<210> 4
   <211> 28
   <212> DNA
   <213> Artificial sequence
<220>
   <223> Synthetic primer
<400> 4
   gttaacagat tggaggtagt agcatttt 28
<210> 5
   <211> 20
   <212> DNA
   <213> Artificial sequence
<220>
   <223> Synthetic primer
<400> 5
   tctatcacca ccccccaact 20
<210> 6
   <211> 27
   <212> DNA
   <213> Artificial sequence
<220>
   <223> Synthetic primer
<400> 6
   caggtatttt ggggtatgat tatggtt 27
<210> 7
   <211> 25
   <212> DNA
   <213> Artificial sequence
<220>
   <223> Synthetic primer
<400> 7
   ccaacagaat ttaccaggaa acaca 25
<210> 8
   <211> 25
   <212> DNA
   <213> Artificial sequence
<220>
   <223> Synthetic primer
<400> 8
   caatacggcc aaatctgaaa gacaa 25
<210> 9
   <211> 21
   <212> DNA
   <213> Artificial sequence
<220>
   <223> Synthetic primer
<400> 9
   ctgcaggttc tccacaccta t 21
<210> 10
   <211> 77
   <212> DNA
   <213> Artificial sequence
<220>
   <223> Synthetic oligonucleotide
<400> 10
   agcagtgctt gctgttcctt agaattttgc cttgtgcgat gctgaatact tgtgataata 60
cacctctacg ggtcagg 77
<210> 11
   <211> 72
   <212> DNA
   <213> Artificial sequence
<220>
   <223> Synthetic oligonucleotide
<400> 11
   ctggttctta ctgcagggcg tgggaggagc gcgatgctga atacttgtga taatacacct 60
ctacgggtca gg 72
<210> 12
   <211> 74
   <212> DNA
   <213> Artificial sequence
<220>
   <223> Synthetic oligonucleotide
<400> 12
   gatagcctgg ggctcactac agacccatga gggcgatgct gaatacttgt gataatacac 60
ctctacgggt cagg 74
<210> 13
   <211> 49
   <212> DNA
   <213> Artificial sequence
<220>
   <223> Synthetic oligonucleotide
<400> 13
   acaaggcaaa attctaagga acagcaagca ctgctgcacg atcaggggt 49
<210> 14
   <211> 46
   <212> DNA
   <213> Artificial sequence
<220>
   <223> Synthetic oligonucleotide
<400> 14
   gctcctccca cgccctgcag taagaaccag accccagcct ttacac 46
<210> 15
   <211> 51
   <212> DNA
   <213> Artificial sequence
<220>
   <223> Synthetic oligonucleotide
<400> 15
   cctcatgggt ctgtagtgag ccccaggcta tctcatgttc ttcagagtgg a 51
<210> 16
   <211> 47
   <212> DNA
   <213> Artificial sequence
<220>
   <223> Synthetic oligonucleotide
<400> 16
   tttttcctga cccgtagagg tgtattatca caagtattca gcatcgc 47
<210> 17
   <211> 32
   <212> DNA
   <213> Artificial sequence
<220>
   <223> Synthetic oligonucleotide
<400> 17
   tttttccgta gaggtgtatt atcacaagta tt 32
<210> 18
   <211> 36
   <212> DNA
   <213> Artificial sequence
<220>
   <223> Synthetic oligonucleotide
<400> 18
   tggatgctga atacttgtga taatacacct ctacgg 36
<210> 19
   <211> 41
   <212> DNA
   <213> Artificial sequence
<220>
   <223> Synthetic oligonucleotide
<400> 19
   tttttccgta gaggtgtatt atcacaagta ttcagcatcc a 41
<210> 20
   <211> 36
   <212> DNA
   <213> Artificial sequence
<220>
   <223> Synthetic oligonucleotide
<400> 20
   ccgtagaggt gtattatcac aagtattcag catcca 36
<210> 21
   <211> 39
   <212> DNA
   <213> Artificial sequence
<220>
   <223> Synthetic oligonucleotide
<400> 21
   ttttttgtca accatcatcg ttcgtaccac agtgttcag 39
<210> 22
   <211> 36
   <212> DNA
   <213> Artificial sequence
<220>
   <223> Synthetic oligonucleotide
<400> 22
   tggatgctga atacttgtga taatacacct ctacgg 36
<210> 23
   <211> 61
   <212> DNA
   <213> Artificial sequence
<220>
   <223> Synthetic oligonucleotide
<400> 23
   ccgtagaggt gtattatcac aagtattcag catccactga acactgtggt acgaacgatg 60
a 61
<210> 24
   <211> 41
   <212> DNA
   <213> Artificial sequence
<220>
   <223> Synthetic oligonucleotide
<400> 24
   tttttccgta gaggtgtatt atcacaagta ttcagcatcc a 41
<210> 25
   <211> 34
   <212> DNA
   <213> Artificial sequence
<220>
   <223> Synthetic oligonucleotide
<400> 25
   ctgaacactg tggtacgaac gatgatggtt gaca 34
<210> 26
   <211> 21
   <212> DNA
   <213> Artificial sequence
<220>
   <223> Synthetic primer
<400> 26
   ccaggggctg tgcagaatgg a 21
<210> 27
   <211> 27
   <212> DNA
   <213> Artificial sequence
<220>
   <223> Synthetic primer
<400> 27
   tctaaaaata gagcatgaaa tggctcc 27
<210> 28
   <211> 28
   <212> DNA
   <213> Artificial sequence
<220>
   <223> Synthetic primer
<400> 28
   gaggtcatgt gcatttaaag ttgggttc 28
<210> 29
   <211> 13
   <212> DNA
   <213> Artificial sequence
<220>
   <223> Synthetic primer
<400> 29
   gcacgccccc ggc 13
<210> 30
   <211> 18
   <212> DNA
   <213> Artificial sequence
<220>
   <223> Synthetic primer
<400> 30
   ggcagctctg gagggcgc 18
<210> 31
   <211> 24
   <212> DNA
   <213> Artificial sequence
<220>
   <223> Synthetic primer
<400> 31
   actaacctgt taacttcgag ctga 24
<210> 32
   <211> 21
   <212> DNA
   <213> Artificial sequence
<220>
   <223> Synthetic primer
<400> 32
   cctgaggtgg gcactggact g 21
<210> 33
   <211> 28
   <212> DNA
   <213> Artificial sequence
<220>
   <223> Synthetic primer
<400> 33
   gtatggtttt gcttaatttc cctaacac 28
<210> 34
   <211> 27
   <212> DNA
   <213> Artificial sequence
<220>
   <223> Synthetic primer
<400> 34
   cttcagcaca aactccttct aagcaga 27
<210> 35
   <211> 24
   <212> DNA
   <213> Artificial sequence
<220>
   <223> Synthetic primer
<400> 35
   gttcgcaggg tttcacaaaa atag 24
<210> 36
   <211> 26
   <212> DNA
   <213> Artificial sequence
<220>
   <223> Synthetic primer
<400> 36
   gagccctact tttgaatccc caggta 26
<210> 37
   <211> 35
   <212> DNA
   <213> Artificial sequence
<220>
   <223> Synthetic primer
<400> 37
   ctgaagcatc ttagtgcttt taaattatag aatta 35
<210> 38
   <211> 38
   <212> DNA
   <213> Artificial sequence
<220>
   <223> Synthetic primer
<400> 38
   accacttaag atttgatttt tatcattaat tgtgatgc 38
<210> 39
   <211> 27
   <212> DNA
   <213> Artificial sequence
<220>
   <223> Synthetic primer
<400> 39
   gtatcagagc caagttcaat aagcatt 27
<210> 40
   <211> 27
   <212> DNA
   <213> Artificial sequence
<220>
   <223> Synthetic primer
<400> 40
   ggaaatgagg ttgggaccaa aagaaga 27
<210> 41
   <211> 20
   <212> DNA
   <213> Artificial sequence
<220>
   <223> Synthetic primer
<400> 41
   atcacctccc ccagatcact 20
<210> 42
   <211> 24
   <212> DNA
   <213> Artificial sequence
<220>
   <223> Synthetic primer
<400> 42
   actgcctgct tgtcaacagc tatc 24
<210> 43
   <211> 26
   <212> DNA
   <213> Artificial sequence
<220>
   <223> Synthetic primer
<400> 43
   acttccatgt tttaaagcat ctacgg 26
<210> 44
   <211> 69
   <212> DNA
   <213> Artificial sequence
<220>
   <223> Synthetic oligonucleotide
<400> 44
   tgccacagcc agaaatcagc gcacggcgcg atgctgaata cttgtgataa tacacctcta 60
   cgggtcagg 69
<210> 45
   <211> 74
   <212> DNA
   <213> Artificial sequence
<220>
   <223> Synthetic oligonucleotide
<400> 45
   gagtctctta ggaccccagg gttctaaggc ccgcgatgct gaatacttgt gataatacac 60
   ctctacgggt cagg 74
<210> 46
   <211> 69
   <212> DNA
   <213> Artificial sequence
<220>
   <223> Synthetic oligonucleotide
<400> 46
   ggtgggaagg agggagcacg ggcttccgcg atgctgaata cttgtgataa tacacctcta 60
   cgggtcagg 69
<210> 47
   <211> 76
   <212> DNA
   <213> Artificial sequence
<220>
   <223> Synthetic oligonucleotide
<400> 47
   cagggttttc tctgattcgt gtgcttctct cccagcgatg ctgaatactt gtgataatac 60
   acctctacgg gtcagg 76
<210> 48
   <211> 72
   <212> **DNA**
   <213> Artificial sequence
<220>
   <223> Synthetic oligonucleotide
<400> 48
   cctccccaca agtgtatgag ggtggctgct gcgatgctga atacttgtga taatacacct 60
   ctacgggtca gg 72
<210> 49
   <211> 79
   <212> DNA
   <213> Artificial sequence
<220>
   <223> Synthetic oligonucleotide
<400> 49
   gcaaggaata acccctgtca aaccatcaga aagaagcgcg atgctgaata cttgtgataa 60
   tacacctcta cgggtcagg 79
<210> 50
   <211> 91
   <212> **DNA**
   <213> Artificial sequence
<220>
   <223> Synthetic oligonucleotide
<400> 50
   cagaaattgt actagatgct gaggactaaa gatacttaag tatcttatcg cgatgctgaa 60
   tacttgtgat aatacacctc tacgggtcag g 91
<210> 51
   <211> 79
   <212> DNA
   <213> Artificial sequence
<220>
   <223> Synthetic oligonucleotide
<400> 51
   tcttccctct tgctttgtca atcagttctc agtagcagcg atgctgaata cttgtgataa 60
   tacacctcta cgggtcagg 79
<210> 52
   <211> 76
   <212> **DNA**
   <213> Artificial sequence
<220>
   <223> Synthetic oligonucleotide
<400> 52
   gggctcaaaa gatggccagc agaagaagca tagtgcgatg ctgaatactt gtgataatac 60
   acctctacgg gtcagg 76
<210> 53
   <211> 78
   <212> DNA
   <213> Artificial sequence
<220>
   <223> Synthetic oligonucleotide
<400> 53
   aaaacaaggc agtcacaaag gctagcacta ccattggcga tgctgaatac ttgtgataat 60
   acacctctac gggtcagg 78
<210> 54
   <211> 77
   <212> DNA
   <213> Artificial sequence
<220>
   <223> Synthetic oligonucleotide
<400> 54
   aatgtctagc ccatagtgga tgccctgtta gtggtgcgat gctgaatact tgtgataata 60
   cacctctacg ggtcagg 77
<210> 55
   <211> 43
   <212> DNA
   <213> Artificial sequence
<220>
   <223> Synthetic oligonucleotide
<400> 55
   gccgtgcgct gatttctggc tgtggcagaa gagatgccta caa 43
<210> 56
   <211> 46
   <212> DNA
   <213> Artificial sequence
<220>
   <223> Synthetic oligonucleotide
<400> 56
   gggccttaga accctggggt cctaagagac tcatcctgaa gccaag 46
<210> 57
   <211> 41
   <212> DNA
   <213> Artificial sequence
<220>
   <223> Synthetic oligonucleotide
<400> 57
   ggaagcccgt gctccctcct tcccaccaca catggcacaa a 41
<210> 58
   <211> 45
   <212> DNA
   <213> Artificial sequence
<220>
   <223> Synthetic oligonucleotide
<400> 58
   tgggagagaa gcacacgaat cagagaaaac cctgcaggga cagca 45
<210> 59
   <211> 41
   <212> DNA
   <213> Artificial sequence
<220>
   <223> Synthetic oligonucleotide
<400> 59
   agcagccacc ctcatacact tgtggggagg gagcctccag c 41
<210> 60
   <211> 55
   <212> DNA
   <213> Artificial sequence
<220>
   <223> Synthetic oligonucleotide
<400> 60
   gcttctttct gatggtttga caggggttat tccttgcaaa caaaacagac caaaa 55
<210> 61
   <211> 65
   <212> DNA
   <213> Artificial sequence
<220>
   <223> Synthetic oligonucleotide
<400> 61
   gataagatac ttaagtatct ttagtcctca gcatctagta caatttctgg ttctttgtat 60
   gcagt 65
<210> 62
   <211> 50
   <212> DNA
   <213> Artificial sequence
<220>
   <223> Synthetic oligonucleotide
<400> 62
   tgctactgag aactgattga caaagcaaga gggaagacct ttctgaggcc 50
<210> 63
   <211> 46
   <212> DNA
   <213> Artificial sequence
<220>
   <223> Synthetic oligonucleotide
<400> 63
   actatgcttc ttctgctggc catcttttga gcccacaggc agggaa 46
<210> 64
   <211> 47
   <212> DNA
   <213> Artificial sequence
<220>
   <223> Synthetic oligonucleotide
<400> 64
   caatggtagt gctagccttt gtgactgcct tgttttgggc tgccatg 47
<210> 65
   <211> 50
   <212> DNA
   <213> Artificial sequence
<220>
   <223> Synthetic oligonucleotide
<400> 65
   accactaaca gggcatccac tatgggctag acattgtcct aagcacttca 50
<210> 66
   <211> 47
   <212> DNA
   <213> Artificial sequence
<220>
   <223> Synthetic oligonucleotide
<400> 66
   tttttcctga cccgtagagg tgtattatca caagtattca gcatcgc 47